# EUROPEAN PATENT SPECIFICATION

(11) **EP 4 455 601 B1**
(45) Date of publication and mention of the grant of the patent: **25.11.2026**
(21) Application number: 24199337.7
(22) Date of filing: 06.06.2019
(51) Int. Cl.: G06F 21/32, G06F 21/45, F41A 17/06, F41A 17/30, A61F 11/08, A61F 11/12, G06K 19/077, A61F 11/14

(54) **WEAPON SYSTEM WITH OPERATOR IDENTIFICATION**
WAFFENSYSTEM MIT BEDIENERERKENNUNG
SYSTÈME D'ARME AVEC IDENTIFICATION DE L'OPÉRATEUR

(30) Priority: 06.06.2018 US 201862681486 P
(43) Date of publication of application: 30.10.2024
(60) Divisional application: 26178190.0 / 4 768 001
(62) Document number(s) of the earlier application(s) in accordance with Art. 76 EPC: 19815047.6 / 3 776 297
(73) Proprietor: Wilcox Industries Corp., Newington, NH 03801 (US)
(72) Inventor: TEETZEL, James W., Portsmouth, NH 03801 (US); LEMIRE, Gary M., Lee, NH 03824 (US); ASSADI, Roula, Stratham, NH 03885 (US)
(74) Representative: FRKelly

(56) References cited:
- US-A- 5 062 232
- US-A1- 2013 127 980
- US-A1- 2014 166 751
- US-A1- 2014 283 429
- US-A1- 2016 054 080

## Description

### CROSS-REFERENCE TO RELATED APPLICATION

This application claims the priority benefit of U.S. provisional application no. 62/681,486 filed June 6, 2018.

### BACKGROUND

United States Patent Application Publication No. US 2014/0283429 A1 discloses a system for precision aiming of a weapon that has a rail aligned with a centerline of its barrel. A bus supplies power to, and provides communication between, a plurality of modular system components that are mounted and precisely aligned in an array on the rail. A core component receives data from the sensor components via the rail and produces an output for display of aiming data which allows an operator to precisely aim the weapon at a selected target.

United States Patent Application Publication No. US 2013/0127980 A1 discloses a near field communication (NFC) device which includes a wrist-worn NFC-enabled electronics device that includes a first communications link for communicating with a second NFC-enabled electronics device via NFC protocols and a second communications link for communicating with an eyepiece via a medium-range communications protocol and receiving control commands. The wrist-worn NFC-enabled electronics device facilitates the transfer of data between the eyepiece and the second NFC-enabled electronics device. The eyepiece comprises optics enabling a see through display for displaying the data.

United States Patent Application Publication No. US 2016/0054080 A1 discloses a safety holster, trigger lock, and utility lock with an electronically actuated locking mechanism. This mechanism is configured to allow the weapon or object to be inserted into the device and engage a feature of the weapon to prevent withdrawal before the mechanism is released. The device also includes a control system with a microcontroller unit that actuates the locking mechanism upon receiving a control signal. The holster body or trigger guard is designed to accept the weapon during insertion. During insertion, the locking mechanism admits the weapon, and once inserted, it engages the weapon's feature to retain it in a locked state. The lock remains engaged until it receives an unlocking control signal from an external device.

### SUMMARY

According to the present invention there is provided a weapon system for a firearm as claimed in claim 1.

In one embodiment the system may include a processor, the processor having an associated memory, and an optical reader, the optical reader configured to receive operator-identification information and transmit the operator-identification information to the processor.

In certain embodiments, the optical reader may be a camera configured to generate image signals.

In certain embodiments, the camera may be configured to detect optically readable indicia.

In certain embodiments, the optically readable indicia may be a bar code. In certain embodiments, the camera may comprise a facial-recognition system responsive to the image signals generated by the camera.

In certain embodiments, the facial recognition system may be controllably coupled to the weapon system.

In certain embodiments, the operator-identification information is selected from the group consisting of facial feature data, iris recognition data, eye vein verification data, ocular feature data, machine-readable indicia.

In certain embodiments, the weapons system further includes an accessory eyewear device.

In certain embodiments, the accessory eyewear device comprises a machine-readable indicia.

In certain embodiments, the accessory eyewear device comprises a near-eye display screen.

In certain embodiments, the near-eye display screen is configured to display one or more of the following: a system menu, sighting system menu options, sighting system statistical information, battery power level, user confirmation status, and user biometric data.

In certain embodiments, the machine-readable indicia is selected from the group consisting of: one dimensional bar code, two-dimensional bar code, and Quick Response (QR) code.

In certain embodiments, the camera is capable of reading the machine-readable indicia.

In certain embodiments, the camera is capable of tracking positioning of at least one eye of an operator.

In certain embodiments, the accessory eyewear device further includes an in-ear communication system, the in-ear communication system comprising an electrical connector and an ear-fitting unit removably attachable to the electrical connector.

In certain embodiments, the electrical connector and the ear-fitting unit are removably attachable by a magnetic force.

In certain embodiments, the control unit is in operable communication with the electrical connector.

In certain embodiments, the ear-fitting unit includes an internal power supply.

In certain embodiments, the ear-fitting unit includes an external power supply.

In certain embodiments, the ear-fitting unit includes an exterior enclosure custom-fitted to a user's ear shape.

In certain embodiments, the ear-fitting unit further includes one or more biological sensors for generating signals representative of biometric information.

In certain embodiments, the biological sensors comprise a means for measuring electrical signals of a user's body.

In certain embodiments, the control unit comprises signal processing circuitry and an audio speaker, wherein the signal processing circuitry is configured to receive the information representative of biometric information and generate an audible notification signal to the audio speaker.

In certain embodiments, the one or more biological sensors are selected from the group consisting of electrodes, temperature transducers, moisture sensors, optical sensors, heart rate sensor, and oxygen saturation sensor.

In certain embodiments, the ear-fitting unit further includes a microphone. In certain embodiments, the microphone is configured to detect acoustic information representative of a user's acoustic signature.

In certain embodiments, the signal processing circuitry comprises analog-to-digital and digital-to-analog conversion circuitry.

In certain embodiments, the control unit further comprises a radio frequency (RF) transceiver.

In certain embodiments, the control unit further comprises a battery power supply.

In certain embodiments, the ear-fitting unit is configured to reduce background noise.

In certain embodiments, the weapon system further includes an external microphone.

In certain embodiments, the signal processing circuitry is configured to generate an audio signal.

In certain embodiments, the audio signal is representative of directional information.

In certain embodiments, the directional information is processed by the signal processing circuitry using sound processing algorithm selected from the group consisting of three-dimensional sound processing algorithm, binaural sound processing algorithm, and head-related transfer function sound processing algorithm.

In certain embodiments, the signal processing circuitry is configured to identify potentially threatening sounds and generate a warning signal.

In certain embodiments, the weapon system further includes an eyewear in operable communication with the ear-fitting unit.

In certain embodiments, the electrical connector is attached to the eyewear.

In certain embodiments, the weapon system further includes a brain computer interface for controlling operation of the weapon system.

In certain embodiments, the weapon system further includes at least one electrode configured to generate brain wave signals, wherein the brain computer interface is configured to monitor and evaluate the brain wave signals.

In certain embodiments, the signal processing circuitry is configured to receive the generated brain wave signals and control operation of the weapon system in response to the generated brain signals.

In certain embodiments, the signal processing circuitry is further configured to generate a weapon system control signal and the RF transceiver is configured to receive the weapon system control signal.

In certain embodiments, the generated brain wave signals are representative of target distance information.

In certain embodiments, the weapons system further includes a pistol grip, the pistol grip housing a safety mechanism in operable communication with the facial recognition system.

In certain embodiments, the weapon system further includes a powered accessory rail interface having an upper accessory rail and a lower accessory rail. A first accessory device is removably attachable to the upper accessory rail and is configured to receive electrical power and data signals over the powered accessory rail interface. A power supply is electrically coupled to the powered accessory rail interface.

In certain embodiments, a second accessory device is removably attachable to the lower accessory rail, the second accessory device configured to receive electrical power and data signals over the powered accessory rail interface.

In certain embodiments, the power supply is a battery box electrically coupled to the lower accessory rail.

In certain embodiments, the weapon system further includes a battery level circuit for determining a charge level of one or more batteries located in the battery box.

In certain embodiments, the weapon system further includes an RF transceiver.

In certain embodiments, the RF transceiver is a Bluetooth transceiver.

In certain embodiments, the weapon system further includes a first RFID reader.

In certain embodiments, the first RFID reader is located in the battery box.

In certain embodiments, the weapon system further includes one or more ammunition magazines, each of the one or more ammunition magazines having an RFID chip readable by the RFID reader, the RFID chip configured to transmit information representative of ammunition type to the first RFID reader.

In certain embodiments, the power supply is housed within a pistol grip of the firearm.

In certain embodiments, the weapon system further includes a remote programming fob configured to transmit one or more user program settings to the weapon system via an RF transceiver.

In certain embodiments, the remote programming fob is programmable with one or more of a computer, laptop, and mobile computing device.

In certain embodiments, the weapon system is programmable via the RF transceiver with one or more of a computer, laptop, and mobile computing device.

In certain embodiments, the weapon system further includes a second RFID reader.

In certain embodiments, the weapon system further includes a tactical glove with a RFID chip readable by the second RFID reader, the RFID chip configured to transmit user-identifying information to the second RFID reader.

In certain embodiments, the weapon system further includes a safety mechanism housed within the pistol grip, the safety mechanism configured to prevent
operation of the firearm when the tactical glove with RFID chip is not in proximity to the second RFID reader.

In certain embodiments, the safety mechanism includes a plunger and a solenoid configured to selectively lock and unlock a trigger mechanism of the firearm.

In certain embodiments, the pistol grip further comprises a battery level circuit for determining a charge level of one or more batteries located within the pistol grip.

In certain embodiments, the pistol grip includes one or more user input devices for controlling operation of the weapon system, the one or more user input devices selected from the group consisting of one or more buttons, a keypad, a rotary encoder, or any combination thereof.

In certain embodiments, the weapon system further includes one or more sensors on the barrel for sensing one or more of: barrel temperature, barrel strain, a projectile being fired, and a velocity of the projectile.

### BRIEF DESCRIPTION OF THE DRAWINGS

The invention may take form in various components and arrangements of components, and in various steps and arrangements of steps. The drawings are only for purposes of illustrating preferred embodiments and are not to be construed as limiting the invention.

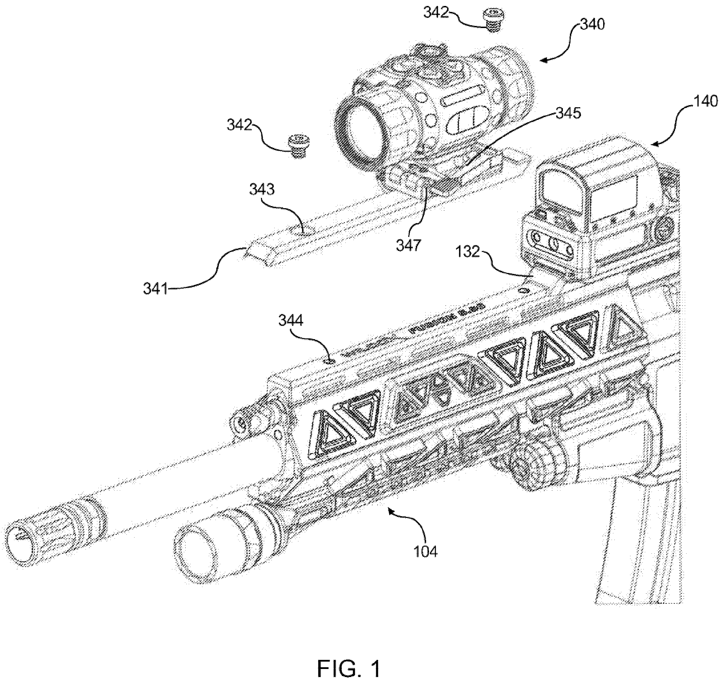

FIG. 1 is an isometric, partially exploded fragmentary view of an exemplary weapon system in a configuration having first and second accessory devices.

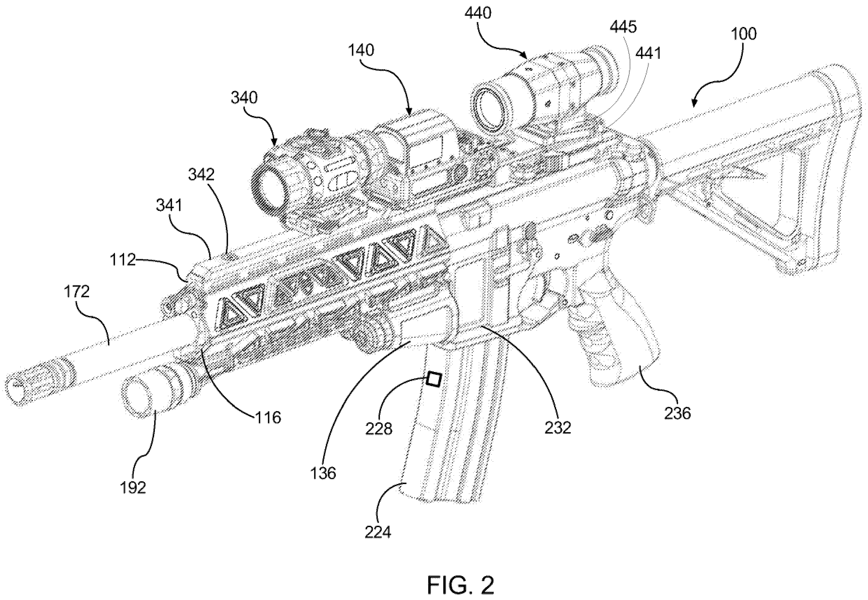

FIG. 2 is an isometric view of the weapon system appearing in FIG. 1, including the first and second accessory devices, and further including an optical scope or magnifier.

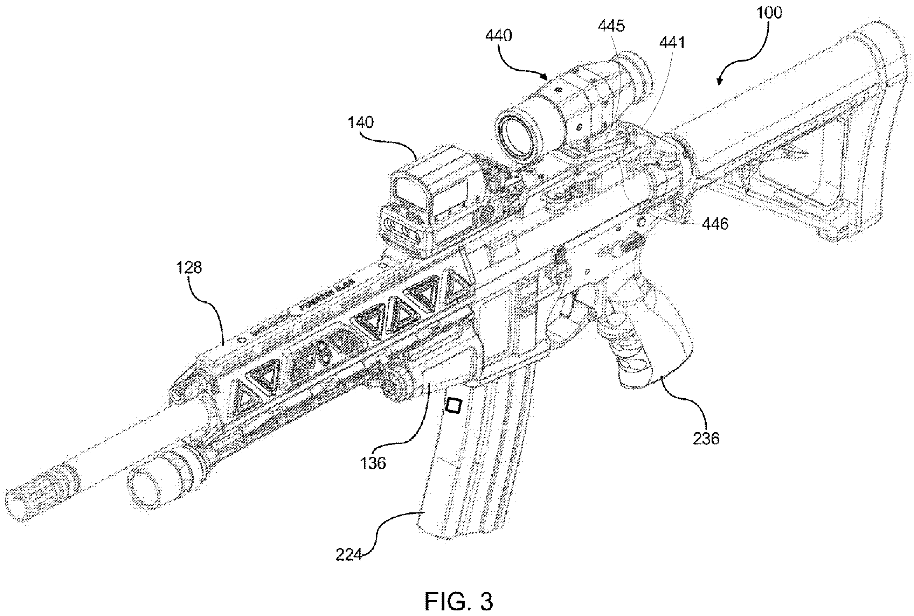

FIG. 3 is an isometric view of the weapon system appearing in FIG. 1, including the first accessory device and the optical scope or magnifier.
FIG. 4 is a fragmentary side view of the weapon system configuration appearing in FIG. 3.

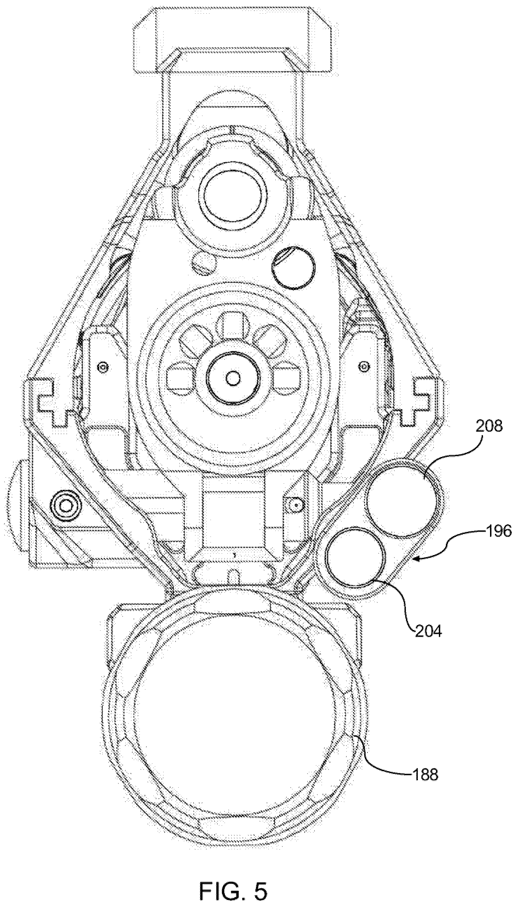

FIG. 5 is a front elevation view of the fore end portion of the illustrated weapon system.

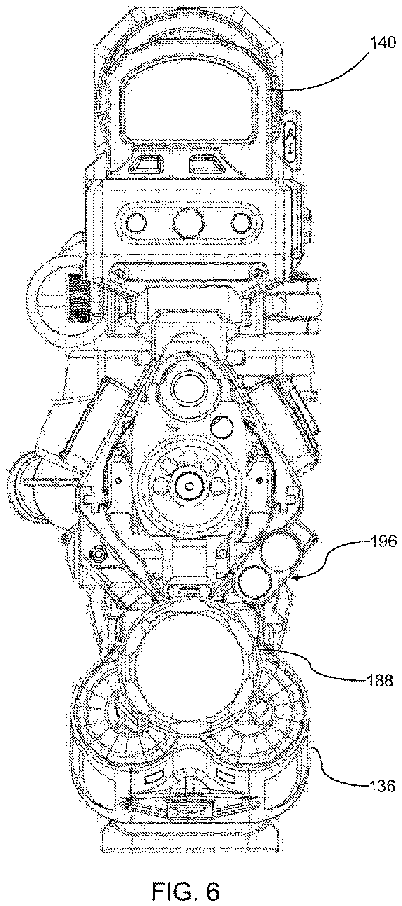

FIG. 6 is a front elevation view of the weapon system configuration appearing in FIGS. 3 and 4 with the magazine removed.

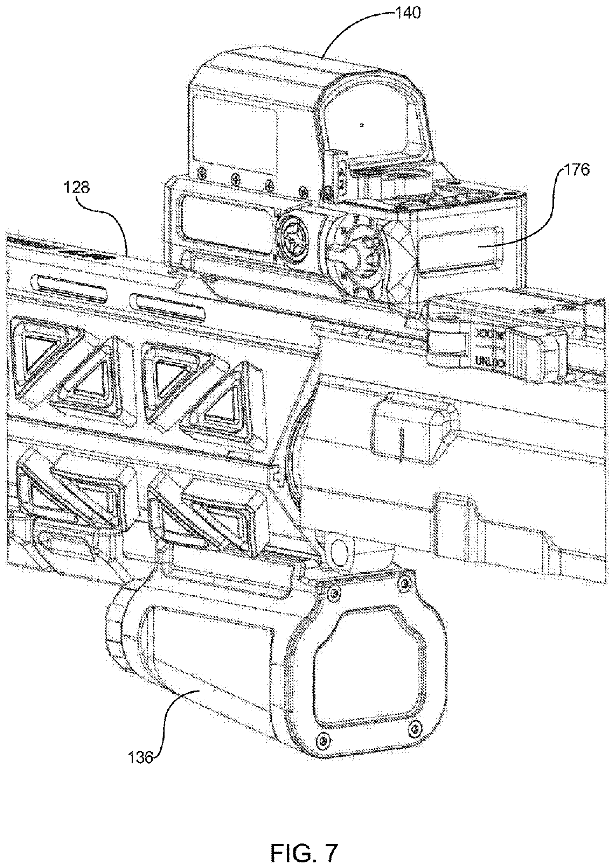

FIG. 7 is a fragmentary isometric view taken generally from the rear and left side.
FIG. 8 illustrates a plurality of interchangeable ammunition magazines operable with the present weapon system.

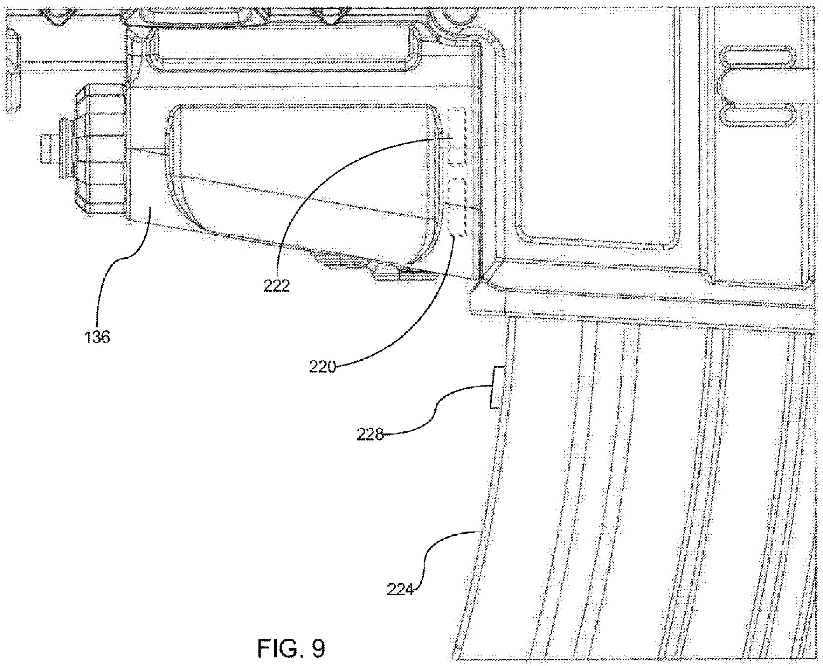

FIG. 9 is an enlarged, fragmentary side elevation view of the weapon system herein having a magazine with RFID tag inserted into the magazine well.

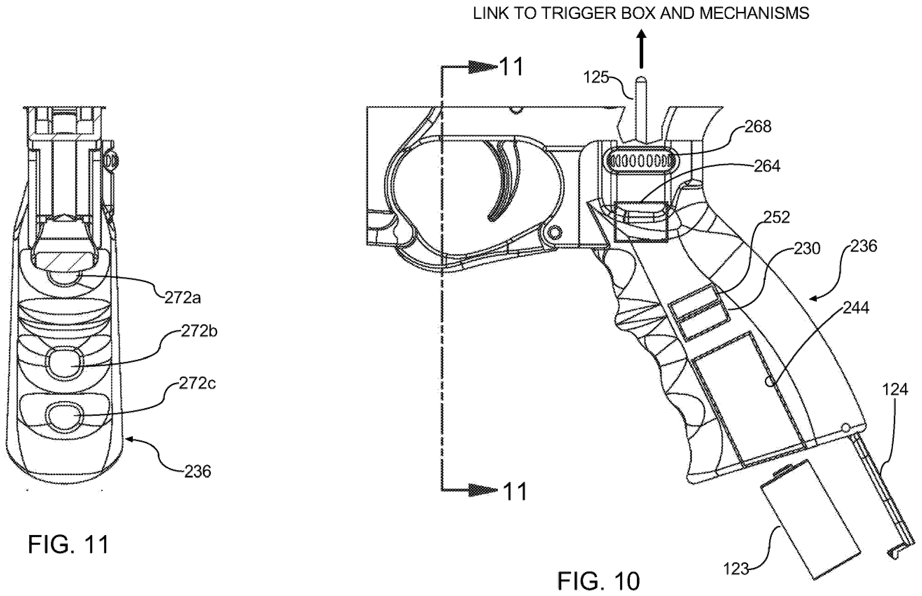

FIG. 10 is an enlarged, fragmentary side view illustrating components of the exemplary weapon system herein within the pistol grip and lower receiver.
FIG. 11 is a cross-sectional view taken along line 11--11 appearing in FIG. 10.
FIG. 12 is a side view of the configuration appearing in FIGS. 3 and 4 and further including a programming fob for programming the weapon system in accordance with the user's preferences or custom settings and one or more mobile devices for programming the fob and/or for programming the weapon system directly.

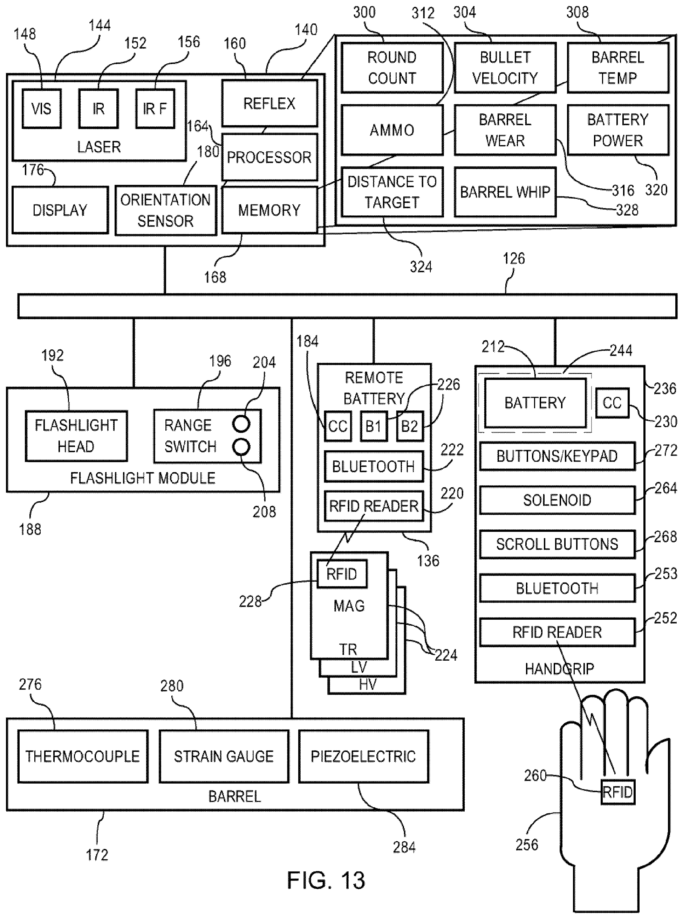

FIG. 13 is a block diagram illustrating an exemplary weapon system in accordance with the present disclosure.

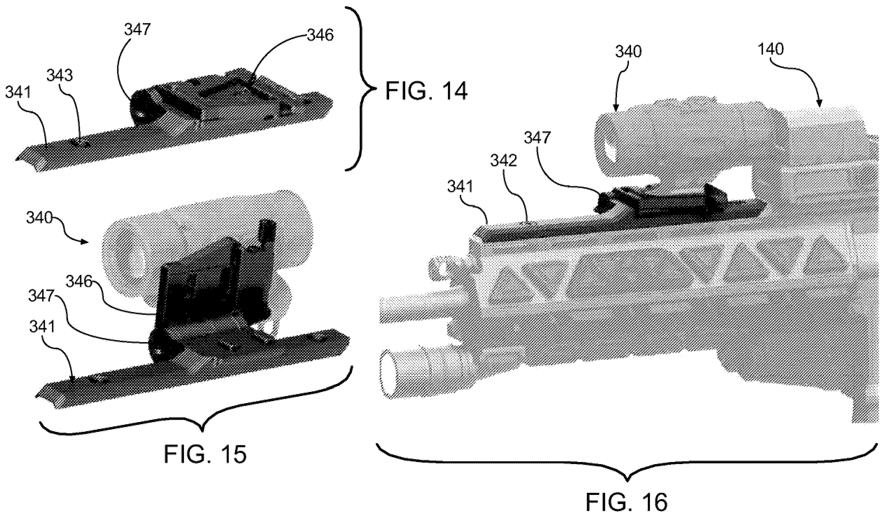

FIG. 14 is an isometric view of the pivot platform.
FIG. 15 is an isometric view of the pivot platform with an accessory device (e.g., camera) attached to the pivot platform and pivoted out of the line of sight.
FIG. 16 is a fragmentary view of the firearm with a second accessory device on the pivot platform in optical alignment with a first accessory device.

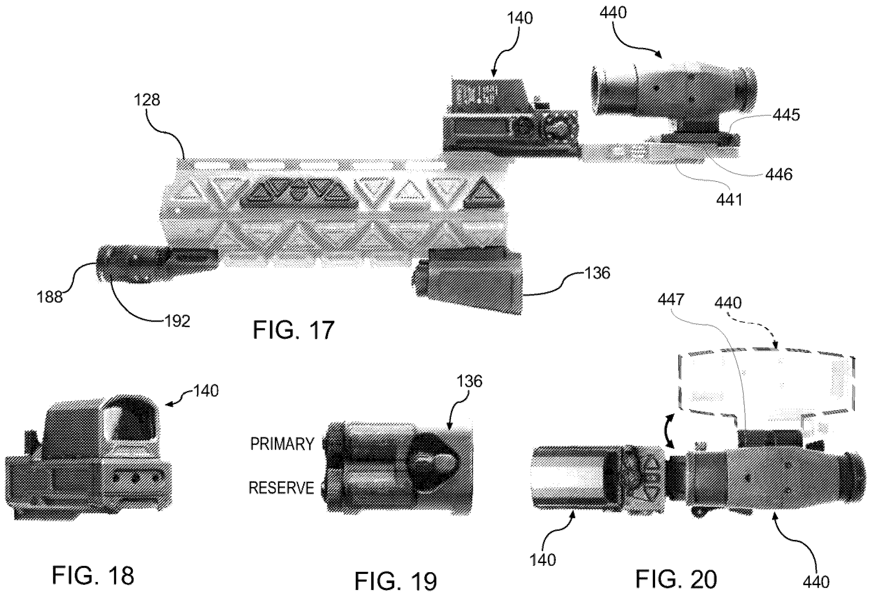

FIG. 17 is a fragmentary side view of the weapon system herein with the firearm and pivot platform removed, and further having a third accessory device (e.g., optical scope or magnifier) positioned behind and in optical alignment with the first accessory device (e.g., laser pointer with integral reflex sight).
FIG. 18 is an isometric view of an exemplary accessory device.
FIG. 19 is a bottom view of an exemplary battery box configured to provide electrical power to an accessory device remotely located on the weapon system.
FIG. 20 is a top view illustrating the first and third accessory devices.
FIG. 21 is a fragmentary isometric view of the configuration appearing in FIGS. and 4 and further including an accessory eyewear device.
FIG. 22 is an isometric view of the accessory eyewear device appearing in FIG. 21.
FIG. 23A is an enlarged, fragmentary side view illustrating components of the exemplary weapon system herein within the pistol grip and lower receiver.
FIG. 23B is a cross-sectional view taken along line23B-23B appearing in FIG. 23A.
FIG. 24 is a side view of the configuration appearing in FIGS. 3 and 4 and further including a programming fob for programming the weapon system in accordance with the user's preferences or custom settings and one or more mobile devices for programming the fob and/or for programming the weapon system directly.
FIG. 25 is a block diagram illustrating an exemplary weapon system including an accessory eyewear device and a tactical glove.
FIG. 26 is an isometric view of the weapon system appearing in FIG. 1, with the second accessory device removed.
FIG. 27 is an isometric view of the weapon system appearing in FIG. 26.
FIG. 28 is an isometric view of the weapon system appearing in FIG. 26.
FIG. 29 is an isometric view of the weapon system appearing in FIG. 26.
FIG. 30 is an isometric view of the weapon system appearing in FIG. 26.
FIG. 31 is an isometric view of the weapon system appearing in FIG. 26 including an accessory eyewear device.
FIG. 32 is an isometric view of the weapon system and accessory eyewear device appearing in FIG. 31.
FIG. 33 is an isometric view of the weapon system and accessory eyewear device appearing in FIG. 31.
FIG. 34 is an isometric view of the weapon system including an accessory eyewear device and tactical glove appearing in FIG. 25.
FIG. 35 is an isometric view of an accessory eyewear device having a pair of ear-fitting units.
FIG. 36 is a rear view of a helmet having the ear-fitting units of FIG. 35.
FIG. 37 is a front view of a headset having the ear-fitting units of FIG. 35.

### DETAILED DESCRIPTION OF THE PREFERRED EMBODIMENTS

Referring now to the drawings, wherein like reference numerals are used to denote like components throughout the several views, the present development relates to a weapon system for use in connection with a weapon such as a firearm **100** and includes a powered accessory rail or platform **104.** In certain embodiments, the powered accessory platform **104** includes a handguard assembly **108** including an upper handguard portion **112** and an opposed lower handguard portion **116,** wherein the upper handguard portion **112** and the lower handguard portion **116** cooperate to define a sleeve, the sleeve having a proximal end configured to attach to the weapon **100** and a distal end opposite the proximal end. The weapon system is configured to sense, receive, and process data to adjust and compensate for weapon performance, for example, to compensate for barrel whip, temperature, harmonic characteristics, etc.

The handguard assembly **108** is configured to surround at least a portion of the weapon **100** when the proximal end is attached to the weapon. An electrical circuit **126** is disposed within the sleeve defined by the handguard assembly **108.** In certain embodiments, the electrical circuit **126** includes a flexible circuit comprising one or more circuit elements, including printed circuit elements, disposed on a flexible circuit substrate. In certain embodiments, the electrical circuit is configured to electrically couple one or more electrically operated devices to a power supply and to provide a data and control signal interface between attached units. A suitable electrical circuit **126** is disclosed in commonly owned U.S. Patent Application Publication No. US2017/0205202 published July 20, 2017 (Ser. No. 15/404,505 filed January 12, 2017).

In certain embodiments, an accessory mounting rail **128** is located on a top portion of the upper handguard portion **112,** the mounting rail **128** having at least one electrical connector thereon. In certain embodiments, an accessory mounting pad **132** is mechanically and electrically coupled to the accessory mounting rail **128** and electrically coupled to a power supply **136** removably attached to the accessory platform **104.** An accessory device **140** is mechanically and electrically coupled to the accessory mounting pad **132.**

In certain embodiments, the powered accessory rail or platform **104** may be of the type disclosed in commonly owned U.S. Patent Application Publication No. US2016/0327371 published November 10, 2016 (Ser. No. 15/146,094 filed May 4, 2016) or the aforementioned commonly owned U.S. Patent Application Publication No. US2017/0205202.

The accessory device **140** is removably attachable to the accessory mounting pad **132** on the accessory platform **104.** In certain embodiments, the accessory device **140** is a laser sight. In certain embodiments, the laser sight includes multiple lasers. In certain embodiments, the laser sight includes an integral reflex sight. In certain embodiments, the laser module may be of the type disclosed in commonly owned U.S. Patent Application Publication No. US2016/0102943 published April 14, 2016 (Ser. No. 14/881,779 filed October 13, 2015).

In certain embodiments, a second accessory device **340** is removably attached to a pivot platform **341** which is located on the accessory mounting rail **128.** In certain embodiments, the pivot platform **341** is removably attached to the accessory mounting rail by means of one or more threaded screws **342,** which are threaded through one or more threaded openings **343** in the pivot platform **341,** corresponding with one or more threaded openings **344** in the accessory mounting rail **128.** The second accessory device **340** may be a camera, such as a thermal camera, complementary metal-oxide-semiconductor (CMOS) image sensor, or short wave infrared camera (SWIR), although other accessory devices are contemplated. The second accessory device **340** includes a dovetail mounting shoe **345** configured to be received in a mounting shoe receiver **346** of the pivot platform. In certain embodiments, the pivot platform is mechanically and electrically coupled to the accessory mounting rail **128** and power supply **136.**

The pivot platform includes a pivot or hinge mechanism **347** which allows the second accessory device **340** to be pivotally adjusted from a first position substantially on top of the pivot platform to a second position substantially to the side of the pivot platform. In certain embodiments, when the second accessory device **340** is in the first position, the second accessory device **340** is coaligned with the first accessory device **140** such that the first and second accessory devices may be used together in single operation. In certain embodiments, the pivot platform includes a lock or clamp device holding the second accessory device **340** in place in the first position. A release mechanism is included such that activation of the release allows the second accessory device **340** to be movable to a second or stowed position. In certain embodiments, when the second accessory device is moved from the second, stowed position to the first, operative position, the second accessory device **340** receives power from power supply **136.** In certain further embodiments, a switch is provided, such that, when the second accessory device **340** is moved from the first, operative position to the second, stowed position, the second accessory device does not receive power from power supply **136.** The switch may be a mechanical switch or a proximity switch, e.g., employing a magnet element and a proximity sensor such as a magnetic reed switch or a Hall effect sensor.

In certain preferred embodiments, the laser sight includes a laser module **144** having one or more lasers, including for example, a visible target pointer laser **148,** an infrared (IR) target pointer laser **152,** and an IR illuminator or flood light laser **156.** In certain embodiments, the lasers **148, 152,** and **156** are factory co-aligned on an optical bench and potted with a potting compound during manufacture to maintain the co-aligned state.

In certain embodiments, the accessory device **140** further includes an integrated reflex sight **160.** In certain embodiments, the reflex sight **160** is co-aligned with the laser module **144.**

In certain embodiments, the accessory device **140** includes a processor **164** and an associated electronic memory **168.** In certain embodiments, the memory **168** includes a program of instructions executed by the processor **164** for performing ballistics calculations based on, e.g., distance to a target, ammunition type, and other factors, by automatically adjusting the sight to assist the user in aligning the barrel **172** of the weapon **100** to achieve a firing trajectory which will cause the path of a projectile fired by the weapon **100** to intersect with the position of a desired target. In certain embodiments, the firing trajectory is adjusted based on the processor's calculations, via a stepper motor and a wedge. The stepper motor selectively advances or retracts a wedge that changes the bias of the laser bench, thereby adjusting the angle of the sight trajectory. One stepper motor/wedge may be provided to provide an elevation adjustment and another stepper motor/wedge may be provided to provide a lateral (windage) adjustment.

In certain embodiments, the trajectory is automatically adjusted based on one or both of (a) the type of ammunition installed; and (b) the distance detected with a range finder component as will be described below.

In certain embodiments, the memory **168** includes a program of instructions executed by the processor **164** for calculating barrel performance.

In certain embodiments, the memory **168** includes a program of instructions executed by the processor **164** for calculating barrel whip.

In certain embodiments, the accessory device **140** includes a human-viewable display **176,** such as an LCD display, LED display, etc., and associated display driver electronics, operably coupled to the processor **164.**

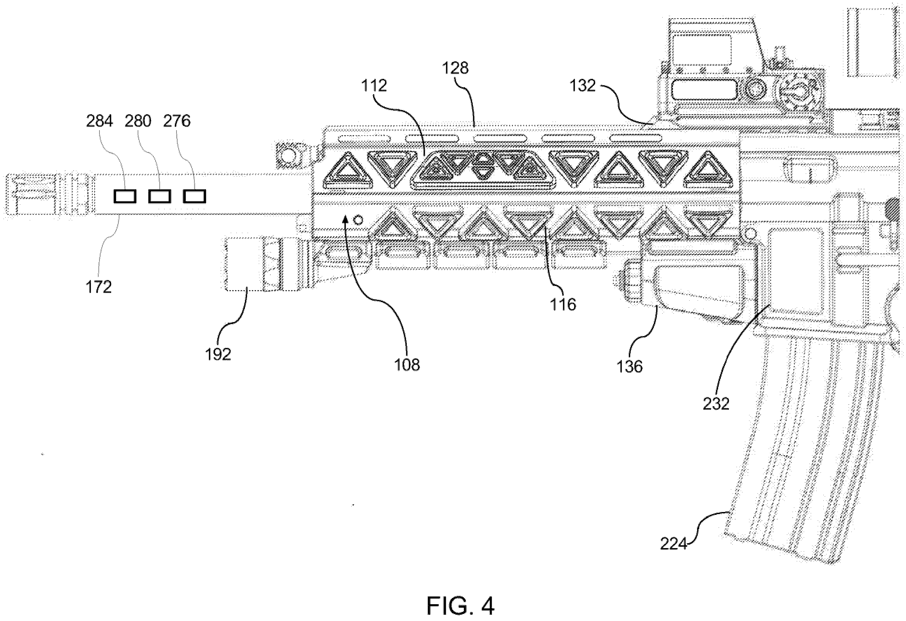

The processor **164** is configured to receive data or signals representative of one or more system parameters, which are logged and stored in the memory **168.** In certain embodiments, the system parameters may be any one or more of the following:
1. Round count based on barrel sensor output (described below),
2. Barrel temperature based on barrel sensor (thermocouple) output.
3. Barrel mileage/life expectancy based on barrel sensor output
4. Rate of fire, e.g., measured in rounds per minute (RPM), based on barrel sensor output.
5. Bullet velocity based on barrel sensor output.
6. Laser mode, e.g., visible, IR, IR Flood, IR+IR Flood.
7. Ammunition cartridge type, including: training rounds (e.g., blanks or non-lethal ammunition rounds), low velocity rounds (e.g., 300 AAC Blackout subsonic rounds), high velocity rounds (e.g., 300 AAC Blackout supersonic rounds), and so forth, based on a radio frequency identification (RFID) tag associated with an installed ammunition magazine.
8. Cant/Tilt function based on an orientation sensor **180,** which may be, e.g., an accelerometer, inclinometer, or the like. A visual indication of the degree of firearm cant or tilt may be output to the display **176** to assist the shooter in leveling the firearm 100 or be used to determine the direction in which the firearm is pointed. For example, aiming direction information may be used in cooperation with a gun lock mechanism, the orientation sensor **180** may be used as an active friend-or-foe system.
9. Battery power remaining (state of charge), e.g., based on output signal from a battery consumption or monitoring circuit **184,** such as a Coulomb counter, battery fuel gauge integrated circuit, voltmeter for measuring battery output voltage or voltage drop due to battery internal resistance, and the like. In certain embodiments, battery state of charge information is stored in a battery power database **320** in the memory **168.** A visual indication of the battery life remaining, e.g., as a percentage based on an estimated battery life, may be output to the display **176.**
10. Barrel whip based on barrel sensor output and barrel harmonic characteristics associated with ammunition cartridge type, barrel length, temperature, and the like. In certain embodiments, barrel information is logged in a barrel whip database **328** stored in the memory **168.** With reference to FIG. 4, bullet/projectile placement varies as a function of (1) the position/alignment of the barrel in relation to the sight that is used to aim the weapon and (2) barrel whip and in particular, the location of the last 120 mm of the barrel as the bullet/projectile exits the barrel. Barrel whip characteristics change as a function of barrel conditions. For example, as the barrel heats up, the barrel droops more, thereby changing the position of the barrel. Shooting the weapon also changes the barrel position. One or more strain gauges monitor such barrel conditions.
11. Distance to target, which may be obtained from a range finder, e.g., an optical range finder integral with the accessory device **140** (not shown) or a separate range finder associated with the weapon system and in communication with the weapon system herein which communication may be via a wired or wireless communication interface. In certain embodiments, the distance to target is logged in a database **324** stored in the memory **168** and may be selectively displayed as an item of information on the display **176.**

In certain embodiments, the distance to target is determined using a rangefinder **196** associated with a flashlight module **188.** The flashlight module **188** is attached at the distal end of the lower handguard member and is electrically coupled to the power supply **136** via the circuit **126.** The flashlight module includes a flashlight head portion **192** and an optical range finder **196.** A mounting member **200** provides a removable electrical and mechanical connection to the lower handguard member **116.**

The flashlight head **192** includes one or more light emitting elements, preferably LEDs. In certain embodiments, the flashlight head **192** includes one or more LEDs which emit radiation in a visible portion of the electromagnetic spectrum. In other embodiments, the flashlight head **192** includes one or more LEDs which emit radiation in an infrared portion of the electromagnetic spectrum. In still further embodiments, the flashlight head **192** is a dual mode flashlight which includes one or more LEDs for selectively emitting radiation in a visible portion of the electromagnetic spectrum and one or more LEDs for selectively emitting radiation in an infrared portion of the electromagnetic spectrum.

In certain embodiments, the range finder **196** comprises a laser emitter **204** and an optical sensor or receiver **208.** The laser **204** sends a beam toward an intended target and reflections of the beam from the target object are detected by the optical receiver **208.** The distance to the target is calculated based on the time-of-flight of the laser beam. In certain embodiments, the laser **204** emits a laser beam in a very short series of pulses, which may be encoded to assist the detector **208** in recognizing the reflected signal.

In certain embodiments, range finder **196** operates as an optical switch to automatically adjust the trajectory of the sight **140** between a "Close Quarter Battle" (CQB) setting and an "Over the Beach" (OTB) setting, under programmed control of the processor **164,** depending on whether the target is within some predetermined threshold value, e.g., some value between 5 and 20 meters, preferably between 5-10 meters, most preferably 10 meters. In certain embodiments, if the trajectory of the sight is set in the OTB setting and the range finder **196** detects that the target is within such predetermined distance, the trajectory of the sight is adjusted to the CQB setting. In certain embodiments, if the trajectory of the sight is set in the CQB setting and the range finder **196** detects that the target is at a distance greater than the predetermined distance, the trajectory of the sight is adjusted to the OTB setting.

Power may be supplied to the accessory device(s) **140** and **240,** flashlight module **188,** or other attached electrically operated devices by one or more of an internal or dedicated power supply for each device, or alternatively or additionally, one or both of the battery box **136** attached to the accessory platform **104** and a power supply **123** contained within a pistol grip **236.** In certain embodiments, the power supply **136** is a battery box as described in the aforementioned commonly owned U.S. Patent Application Publication No. US2017/0205202.

The power supply(ies) includes one or more batteries. In certain embodiments, the power supply includes an RFID reader. In certain embodiments, the power supply **136** includes an RF transceiver interface, such as a Bluetooth transceiver **222.** In certain embodiments, the handgrip **236** includes an RF transceiver interface, such as a Bluetooth transceiver **253.**

In certain embodiments, a third accessory device **440** is removably attached to an ear pivot platform accessory mount, **441,** which is located on the firearm upper receiver behind the pivot platform **341.** In certain embodiments, the rear pivot platform **441** is removably attached to the firearm upper receiver by means of one or more threaded fasteners (not shown), which engage one or more aligned threaded openings in the rear pivot platform **441** and a corresponding one or more threaded openings in the firearm upper receiver. The third accessory device **440** may be an optical device such as an optical magnifier or optical scope. In certain embodiments, the third accessory device **440** is an optical magnifier, e.g., a 3x or 4x optical magnifier, to provide an enlarged view of the integrated reflex sight. The third accessory device **440** includes a dovetail mounting shoe **445** configured to be received in a mounting shoe receiver **446** of the rear pivot platform **441.** In certain embodiments, the rear pivot platform is mechanically and electrically coupled to the accessory mounting rail **128** and power supply **136.**

The rear pivot platform includes a pivot or hinge mechanism **447,** which allows the third accessory device **440** to be pivotally adjusted from a first position in optical alignment with the first accessory device **140** wherein to a second position wherein the third accessory device **440** is moved out of optical alignment with the first accessory device **140.** In certain embodiments, when the third accessory device 340 is in the first position, the third accessory device **440** is coaligned with the first accessory device **140** such that the first and second accessory devices may be used together in single operation. Similarly, in certain embodiments wherein both the second accessory device **340** and the third accessory device **440** are attached and in their respective first or operative positions, the first, second, and third accessory devices **(140, 340, 440)** are coaligned with the first accessory device **140** such that the first, second and third accessory devices may be used together in single operation.

In certain embodiments, the pivot platform includes a lock or clamp device holding the third accessory device **440** in place in the first position. A release mechanism is included such that activation of the release allows the third accessory device **440** to be movable to the second, stowed position.

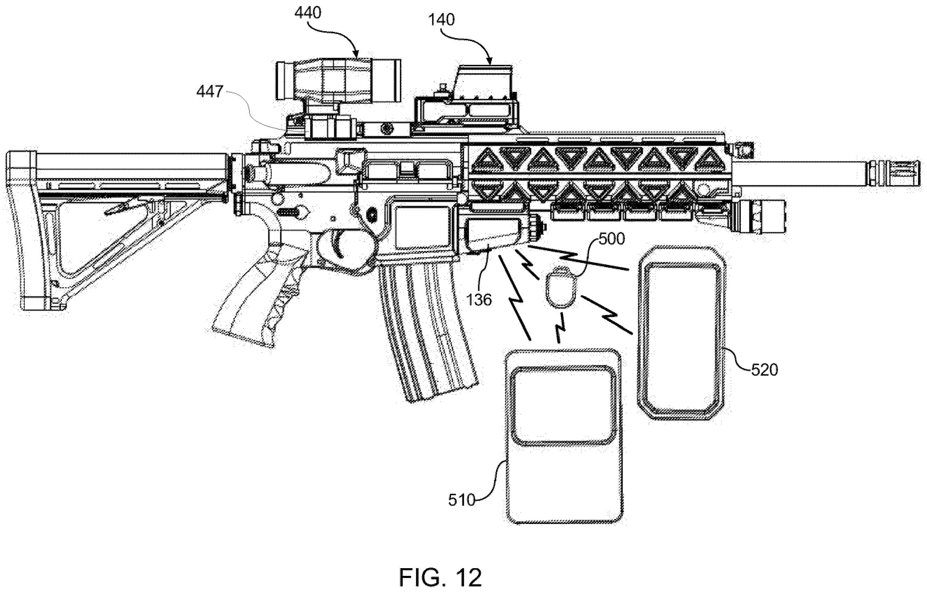

As illustrated in FIG. 12, certain embodiments of the weapon system includes a fob, e.g., key chain type fob, or other portable associated device **500,** that is programmable to store user customizable device settings. This avoids the need for the user to have to take the time to go through all the programming steps to customize it to the person. For example, a soldier may pick up another soldier's rifle that has different and quickly change it over to his or her settings. Thus, the soldier only needs to worry about picking up a gun and shooting it without the confusion or delay of programming. The cell phone or other mobile device, through an app, can do the programming off line, and then transfer it to the battery box controller, either directly or through a programmable key fob.

The fob **500** includes an RF transceiver interface, such as a Bluetooth transceiver. The fob **500** may be remotely and wirelessly programmed, for example, through a fob programming unit, which may be a dedicated fob programming device **510** or a mobile device such as a cell phone, smart phone, handheld data terminal, etc., to configure and store desired weapon systems settings. The battery box **136** includes a transceiver which is capable of receiving a radio frequency signal emitted by the pre-programmed fob **500.** When brought within range of the battery box, the pre-programmed fob **500** transmits the stored custom user settings from the fob **500** to the weapon system and programs the weapon system with the desired settings. In certain embodiments, the fob **500** includes an actuator button for transmitting the settings to the battery box transceiver. In certain embodiments, as an alternative to using the fob **500,** the programming device **510** or alternatively the cell phone, smart phone, or hand held data terminal **520,** etc. running an application program, is programmed user and is then used to transmit the custom program information or settings directly via the Bluetooth transceiver in the battery box without the fob **500.** In certain embodiments, multiple interchangeable magazines **224** configured for holding different types of ammunition cartridges are provided. In certain embodiments, each magazine **224** has an RFID tag **228** attached thereto. The RF tag **228** may be active, semi-active, or passive and contains stored data representative of the type of ammunition cartridges stored within the magazine **224.** In the illustrated embodiment, the RFID reader **220** in the battery box **136** sends signals to and receives signals from the RFID tag **228.** However, it will be recognized that the associated RFID reader may be disposed elsewhere, such as on the firearm upper or lower receiver or on the accessory platform 104 or other device attached to the firearm.

The RF reader **220** and RFID tag **228** are capable of sending and receiving electromagnetic signals to and from each other, thereby allowing the reader to interrogate the tag **228** and obtain data representative of the type of ammunition rounds stored in the magazine that is currently inserted into the magazine well **232** of the firearm **100.** In certain embodiments, the RFID tag 228 is a passive RF tag which does not initiate communications with the RFID reader **220,** but must be read, i.e., wherein the RFID tag **228** utilizes power from the RF waves transmitted by the RFID reader **220.** In preferred embodiments, the communication range between the RFID reader **220** and the RFID tag **228** is in the range of 0 to 1 meter, preferably 0 to 30 centimeters, and more preferably 0 to 20 centimeters.

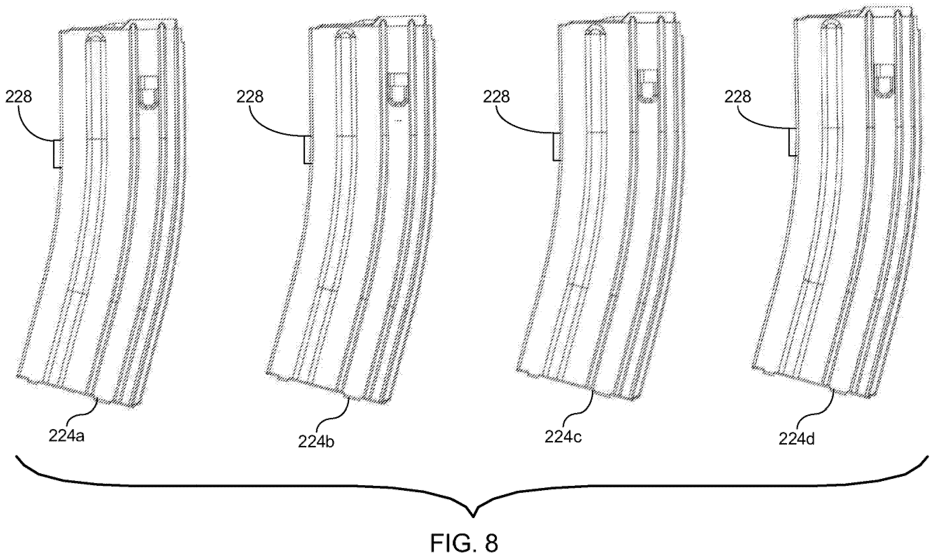

In certain embodiments, as shown in FIG. 8, first one of the magazines **224a** is a magazine for low velocity rounds such as low velocity (subsonic) .300 AAC Blackout rounds, and a second one of the magazines **224b** is a magazine for high velocity (supersonic) .300 AAC Blackout rounds, a third one of the magazines **224c** is a magazine for saboted rounds, e.g., Sabot Light Armor Penetrator (SLAP) rounds such as .300 ACC Blackout SLAP rounds, and a fourth one of the magazines **224d** is a magazine for training rounds. It will be recognized that other ammunition types are also contemplated.

In certain embodiments, the ammunition type is logged in an ammunition database **312** stored in the memory **168** and may be displayed to the user on the display **176,** e.g., as an item of information accessed by the user using scroll buttons **268** on the handguard assembly **108,** as described below.

In operation, the RFID reader **220** reads the data representative of the round type contained on the RFID tag **228** of the installed magazine **224.** The data is transmitted via the accessory platform **128** and the circuit **126** to the accessory device **140** and stored in the memory **168.** In certain embodiments, environmental sensors **501** collect data regarding various environmental parameters, such as outside temperature, directional orientation, etc. The environmental data is transmitted to the processor **164** and may be stored in the memory **168.** The processor **164** then calculates the sight trajectory based on ballistics information for the detected ammunition type as well as other factors, such as the distance to the target as determined using the range finder **196** as described above. In certain embodiments, the firing trajectory is adjusted based on the processor's calculations, via a stepper motor selectively advancing and retracting a bearing against the optical bench of an aligned sighting device.

In certain embodiments, when the installed magazine is a training round, the aiming mark from the reflex sight is configured to appear as a green dot. In certain embodiments, when the installed magazine is a non-training round, the aiming mark from the reflex sight is configured to appear as a green dot. It will be recognized that other reticle shapes, such as rings, cross hairs, and the like, are also contemplated.

In certain embodiments, the handgrip **236** further comprises a power source **123.** One or more batteries **123** are received within an interior battery compartment **244** defined in the handgrip **236.** In certain embodiments, the handgrip includes a battery cap **124.** In certain embodiments, the handgrip **236** is coupled to an RF transceiver, such as the Bluetooth transceiver **222.** Circuitry within the compartment **244,** which may include circuit elements printed circuit elements on a flexible substrate, electrically couple the terminals of the battery(ies) to circuit within the accessory platform to provide battery power and control signals to attached devices, such as the laser sight **140** and/or camera **340.** In certain embodiments, a Coulomb counter or like circuit **230** is provided to output battery state of charge information to the device **140** via the circuit **126.**

In certain embodiments, the handgrip **236** includes an RFID reader **252** for sensing the proximity of a tactical glove **256** carrying an RFID chip tag **260.** In certain embodiments, the handgrip **236** includes an RF transceiver, such as a Bluetooth transceiver **253,** allowing the handgrip **236** to couple with and communicate with the battery box **136,** for example, to activate the weapon system. In certain embodiments, the proximity of the tactical glove is required to activate the weapon system. Alternatively, in certain embodiments, the weapon system includes a solenoid switch **264** operable to selectively engage a trigger lock when the glove **256** is not in proximity to the RFID reader **252** and to disengage the trigger lock when the glove **256** is in proximity to the RFID reader **252.** In certain embodiments, the solenoid switch engages the trigger lock via a pin or plunger **125,** which is operably connected to the trigger mechanisms, such that when the pin is inserted into the trigger mechanism, the trigger cannot be activated.

In certain embodiments, a rotary encoder **268** on the handguard **108** is electrically coupled to the laser sight via the accessory platform **128** is manually rotatable to allow the user to scroll through pages, menus, or other items of viewable information on the display **176.** In alternative embodiments, scroll buttons may be used in placed of the rotary wheel, to scroll through pages, menus, or other items of viewable information on the display.

In certain embodiments, one or more user-programmable switches **272,** e.g., configured as user-depressible buttons, are provided on the handgrip **236.** Signals from the switches **272** are transmitted to the processor **164** via the accessory platform **104** for controlling operation of, for example, the laser sight **140,** flashlight module **188,** push-to-talk radio, and/or other device attached to the accessory platform **104.** Exemplary functions which can be executed on the laser sight **140** using the buttons **272** include any one or more of laser selection, laser actuation, display operation, and navigation of a hierarchal menu structure or other graphical user interface on the display **176.** In certain embodiments, the switches **272** include a first switch **272a,** second switch **272b,** and third switch **272c,** which are user-programmable to allow the user to customize the function of each button.

In certain embodiments, the handgrip housing has an outer material having a good grip adhesion, such as silicone, and may have a textured or contoured surface for enhancing grip. The outer material may be flexible and resilient and disposed over the depressible push button switches **272.**

In certain embodiments, the handgrip **236** is coupled to an RF transceiver, such as the Bluetooth transceiver **222,** to allow the user to control operation of an associated device having a paired RF transceiver, such as a cell phone, smart phone, hand held data terminal, wearable device, or the like via the RF interface using one or more controls on the handgrip, such as one or more buttons or keys **272,** the scroll buttons **268,** and so forth. In certain embodiments, one or more of the buttons **272** actuate a push-to-talk function of an associated two-way communication system, e.g., via the Bluetooth transceiver **222.** In certain embodiments, the associated device includes software for use in identification verification of the user to activate the weapon system.

Firearm barrel sensors on the firearm barrel **172** send signals representative of barrel conditions to the processor **164** via the circuit **126.** In certain embodiments, the sensors include one or more temperature sensors **276,** such as one or more thermocouples, for providing output signals representative of the temperature of the barrel **172.** In certain embodiments, the sensors include one or more strain gauges **280** for providing output signals representative of the degree of mechanical stress or deformation that the barrel is undergoing, e.g., responsive to a round of ammunition being fired by the firearm **100.** In certain embodiments, the sensors include one or more piezo electric sensors **284** for providing output signals representative of the degree of mechanical stress or deformation that the barrel is undergoing, e.g., responsive to a round of ammunition being fired by the firearm **100.**

Exemplary barrels having sensors thereon are disclosed in commonly owned U.S. provisional application no. 62/446,222 filed January 13, 2017, and commonly owned U.S. provisional application no. 62/513,738 filed June 1, 2017.

In certain embodiments, bullet projectile velocity is calculated by the processor **164,** e.g., using the sensors **280** and **284.** In certain embodiments, bullet velocity information is logged in a bullet velocity database **304** stored in the memory **168** and may be displayed to the user on the display **176,** e.g., as an item of information accessed by the user using the scroll buttons **268.**

In certain embodiments, barrel temperature is calculated by the processor **164,** e.g., using the sensor **276.** In certain embodiments, barrel temperature information is logged in a barrel temperature database **308** stored in the memory **168** and may be displayed to the user on the display **176,** e.g., as an item of information accessed by the user using the scroll buttons **268.**

In certain embodiments, signals from the strain gauges **280** and/or piezo electric sensors **284** are sent to the processor for signal processing and/or data storage. It will be recognized that the processor **164** may include and is intended to encompass associated signal processors such as analog-to-digital converters, digital signal processors, and the like, which may be implemented as functional modules within a single, e.g., general purpose, processing unit or as discrete or dedicated hardware processors.

In certain embodiments, the system continuously monitors the barrel sensors **276, 280, 284.** Signal parameters indicative of a round being fired are detected and logged in a round count database **300** stored in the memory **168.** The number of rounds fired may be displayed to the user on the display **176,** e.g., as an item of information accessed by the user using the scroll buttons **268.**

The firing of a round is a wear-creating event. In certain embodiments, each round that is fired is associated with an incremental unit of barrel wear. Each incremental unit of barrel wear is logged in a barrel wear database **316** stored in the memory **168.** The accumulated wear is compared to a preselected wear limit, which may be, for example, the barrel wear (or estimated or predicted barrel wear) that is associated with a certain degree of accuracy loss. The accumulated barrel wear may be displayed in the display **176** and may be indicated in a number of ways, for example, as a percentage of barrel life remaining, calculated as: $100 \times \left(1-accumulated wear/wear limit\right)$ or, alternatively, as a percentage of barrel life consumed: $100 \times accumulated wear / wear limit$

A basic embodiment of the system may base the wear accumulation based solely on the number of rounds fired. In alternative embodiments, the severity of the wear created can be estimated based on other system parameters, such as the barrel temperature, which may be monitored and logged at periodic intervals in the barrel temperature database **308,** or ammunition type, which may be read by the RFID reader **220** and logged in an ammunition database **362** in the memory **168.**

For example, rounds fired when the barrel is at a high temperature generate increments of barrel wear that are more severe than rounds fired when the barrel is not at a high temperature. Similarly, rounds that have increased quantities of primer and propellant/powder (e.g., high velocity rounds) generate increments of barrel wear that are more severe than rounds that have lower amounts of primer and propellant/powder (e.g., low velocity rounds). The severity of barrel wear is affected by the type of primer and propellant/powder in the round.

In certain embodiments, the system estimates the incremental wear associated with firing each round and quantifies the severities based on round type, barrel temperature, rate of fire, and so forth, which provides an improved estimate of barrel wear over wear estimates based solely on the number of rounds fired. In certain embodiments, the severity of wear can be further estimated based on predicted interactions of firearm parameters with each other and/or with interactions of firearm parameters and the current accumulated barrel wear. In certain embodiments, the estimated severity of a given firing event is used to denominate the incremental wear associated with each firing event into units of normalized rounds fired and, as rounds are fired, wear increments in normalized units are added to the preexisting cumulative barrel wear stored in the wear database **316.**

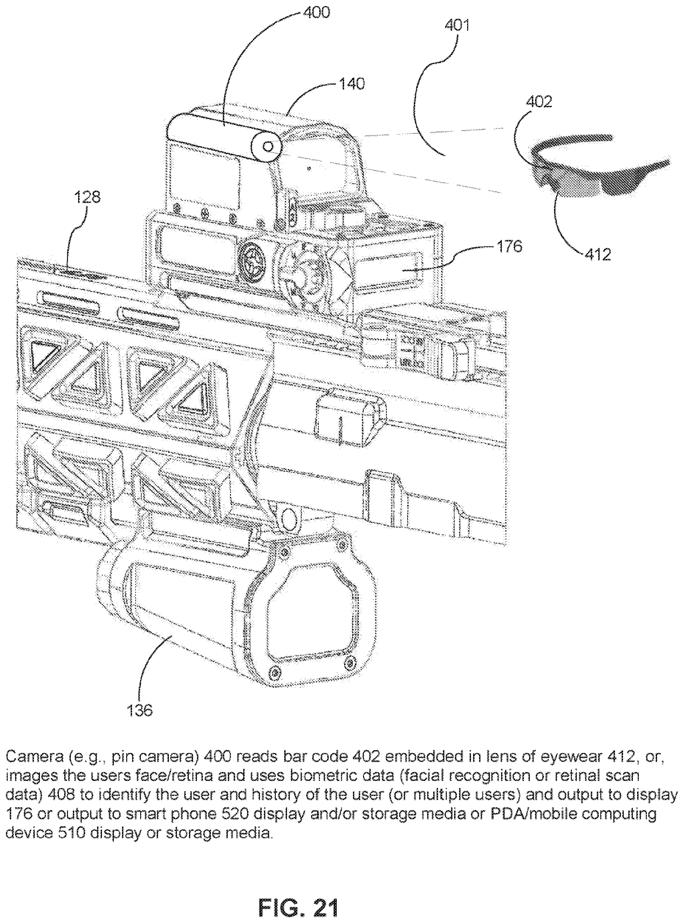

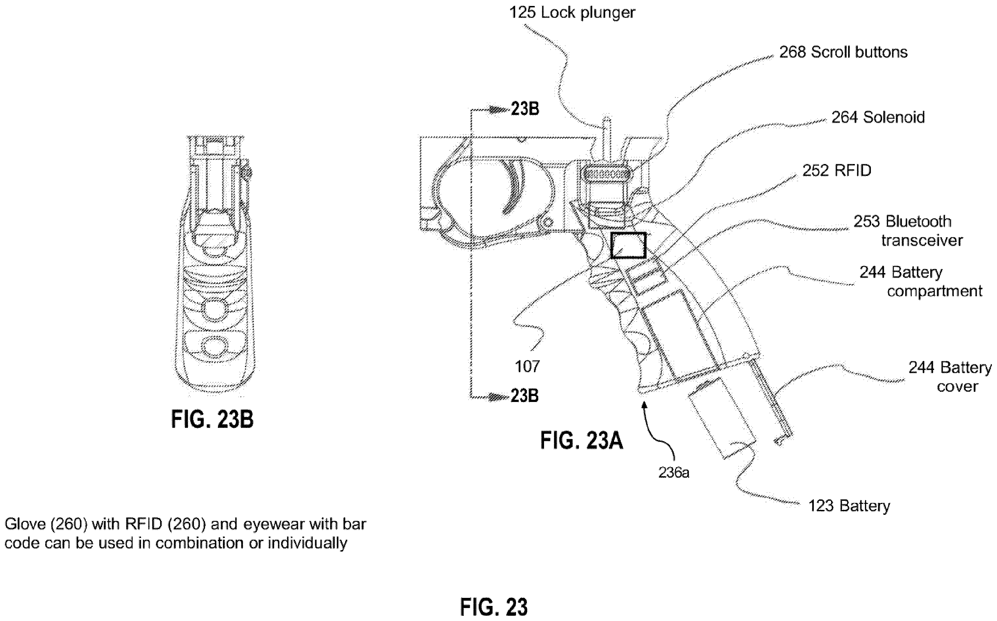

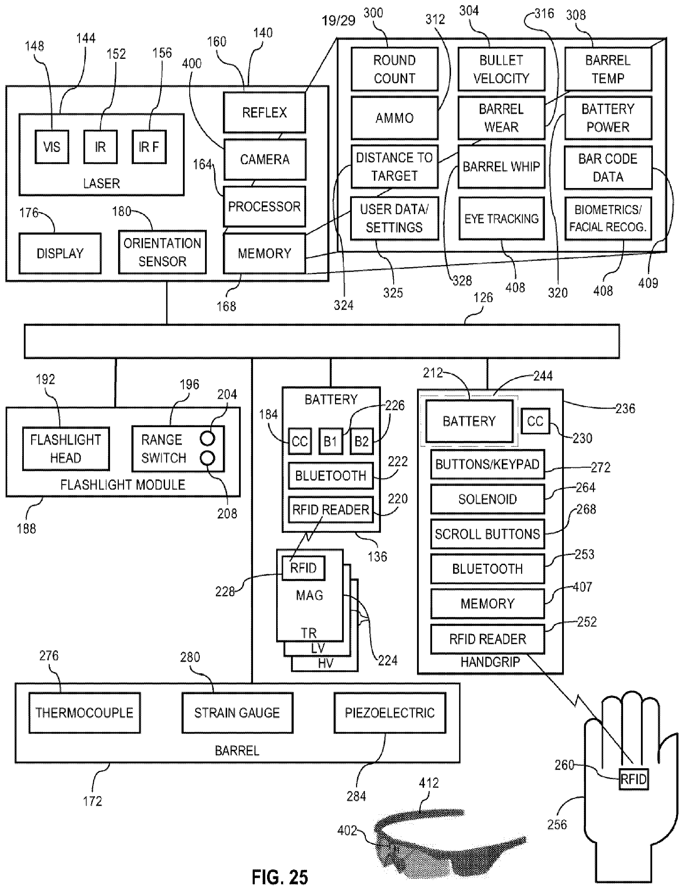

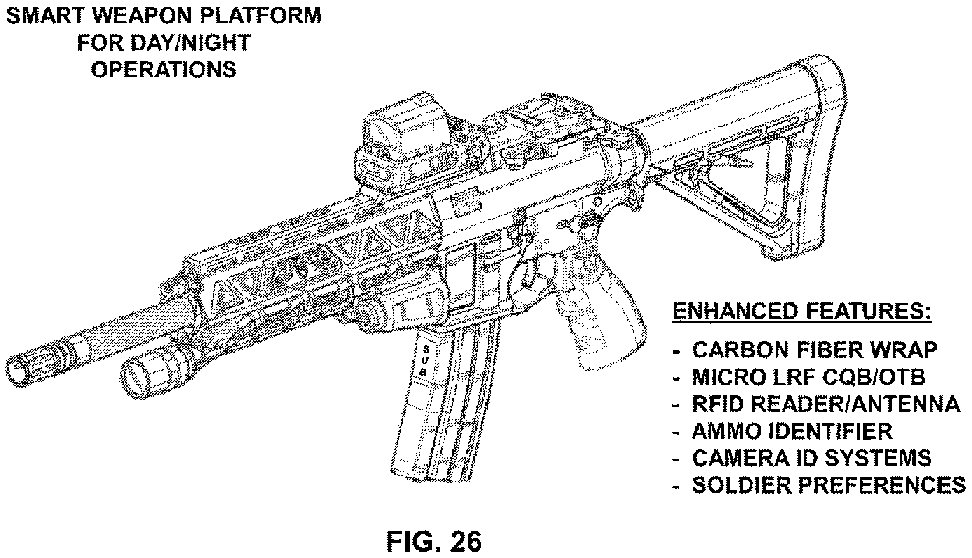

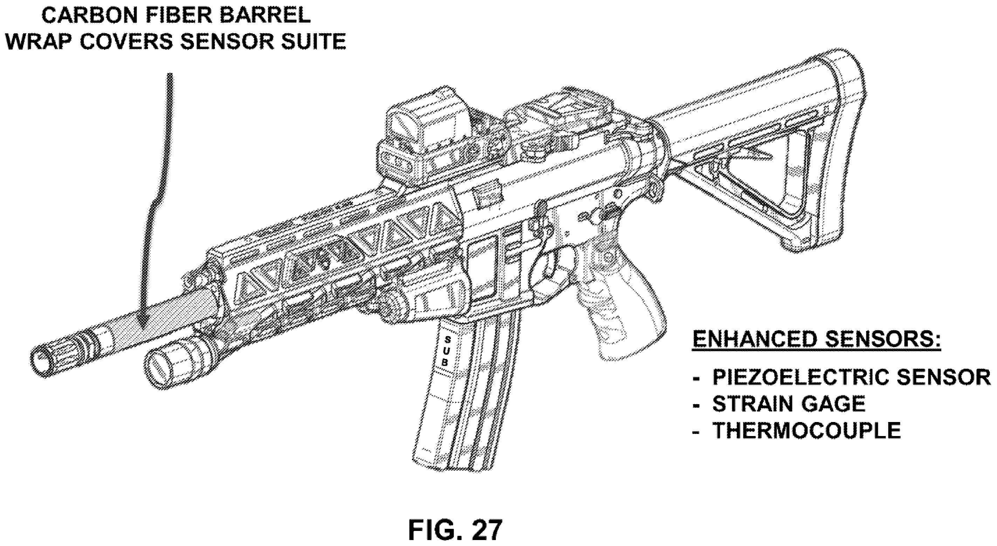

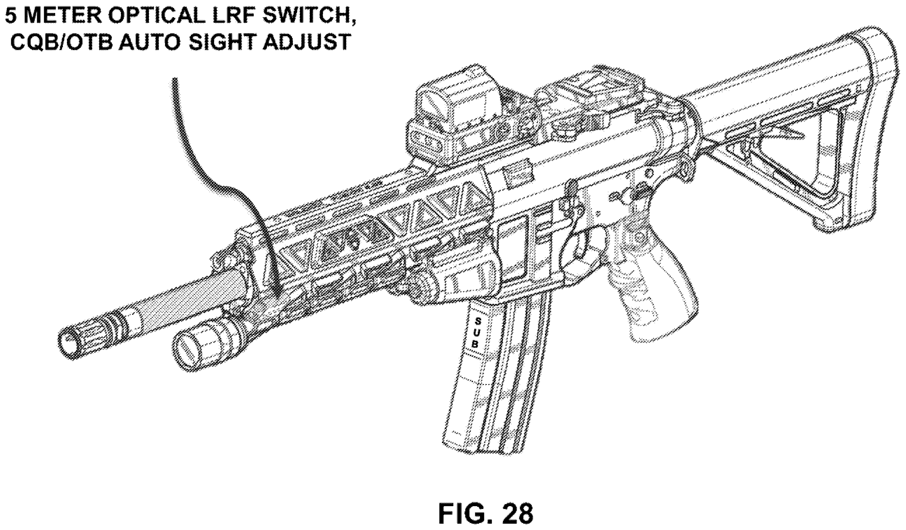

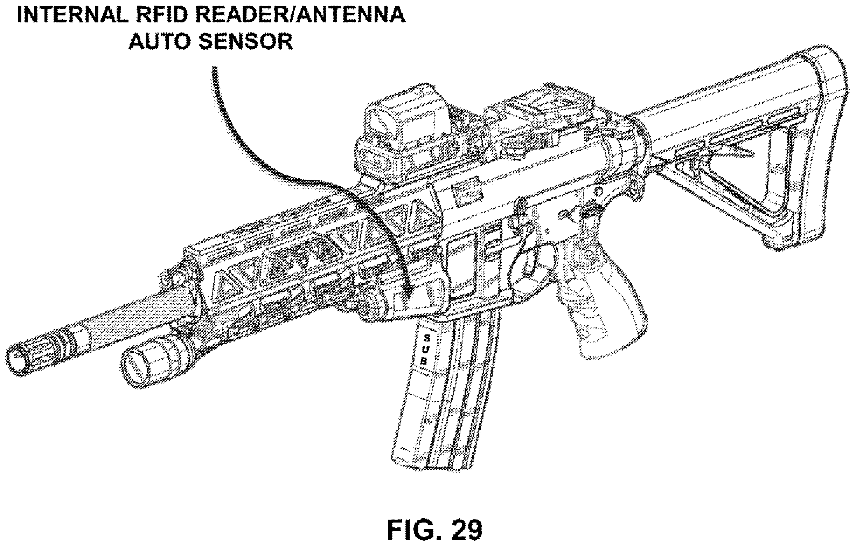

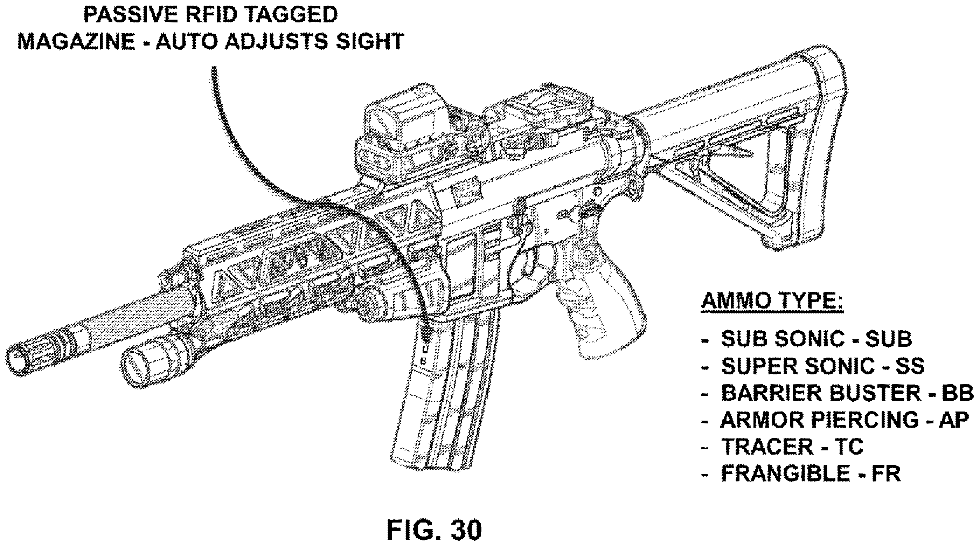

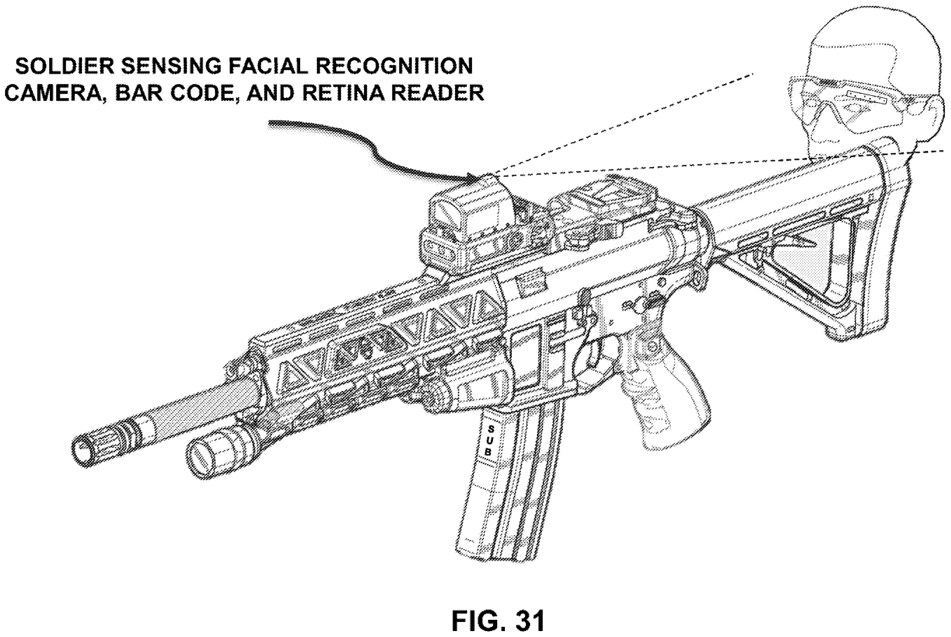

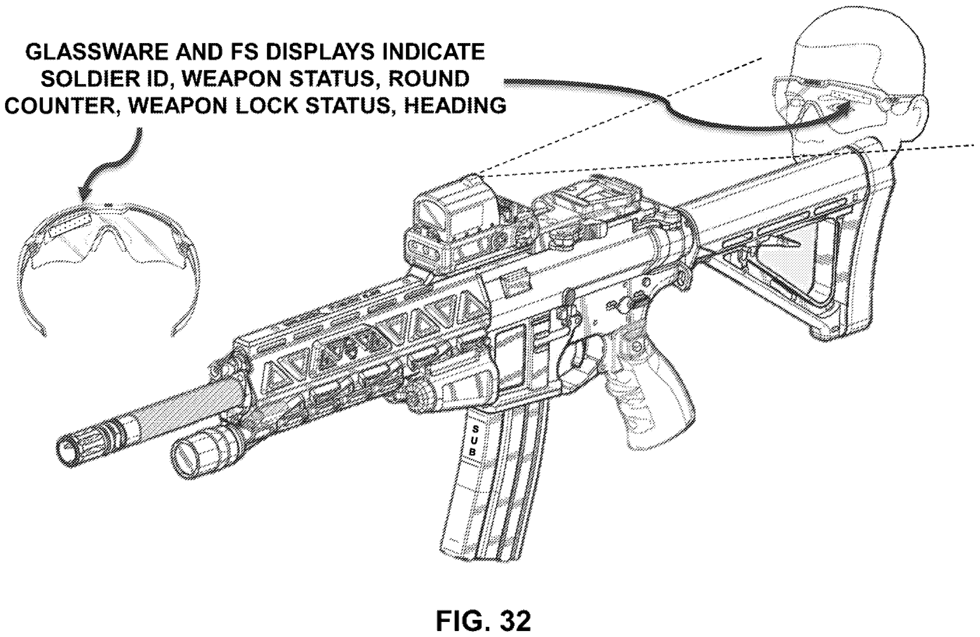

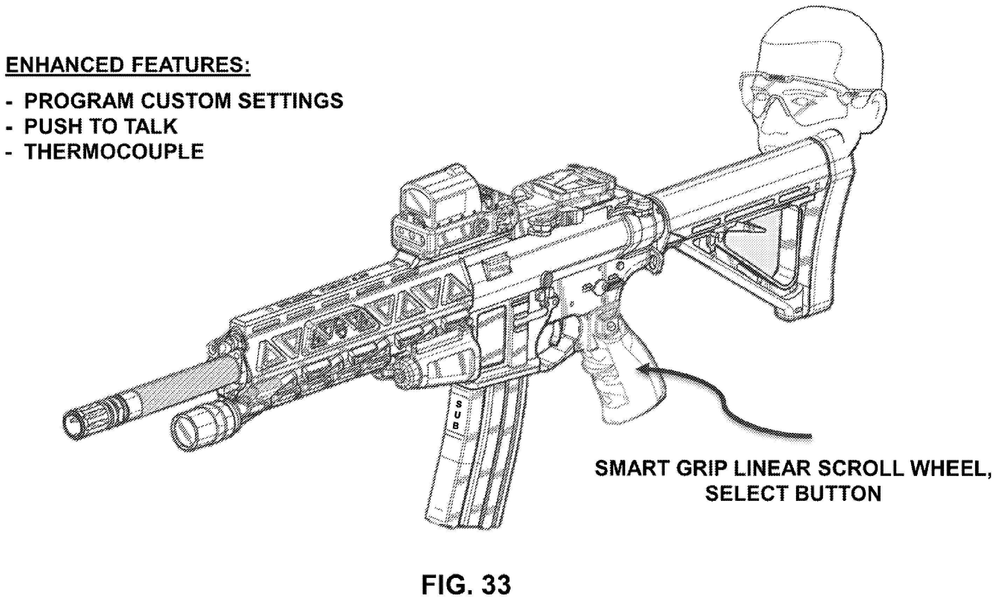

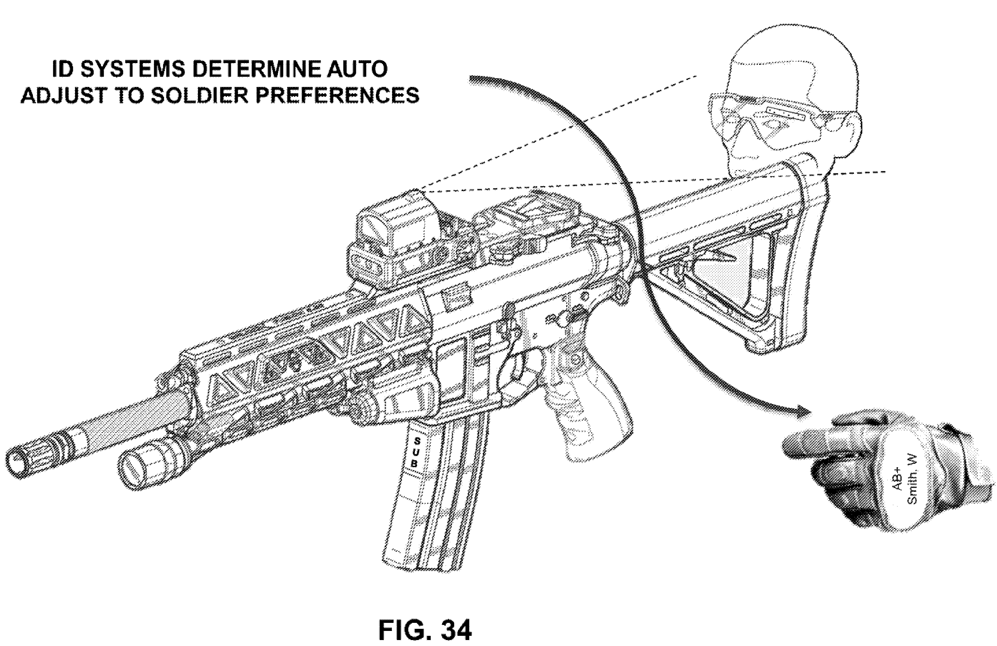

Referring now to FIGS. 21-34, there is illustrated a further embodiment system comprising a laser sight accessory device **140** attached to the accessory rail **128,** the accessory device further having an integrated camera **400,** such as a pin hole camera. The camera **400** is rear facing and is configured to image a field of view **401** which encompasses at least the user's eye region and, in certain embodiments, the user's facial region. The accessory device may be a BOSS(TM) system available from Wilcox Industries Corp. of Newington, NH.

The rear facing camera **400** is located on the top of the unit **140** and camera serves multiple purposes, including: (1) reading a bar code or other optically readable indicia on associated eyewear **412** worn by the user for identification of the operator; (2) performing facial recognition of the operator for user verification/authentication purposes; (3) tracking the positioning of the operator's eye or eyes relative to the weapon to verify what the operator is viewing during operation of the weapon, and (4) allowing the operator to control operation of the unit **140** to perform a ranging function and menu activation and selection via optical interaction with the system.

The eyewear **412** feature a bar code or other optically readable indicia **402** and an integrated near-eye display screen **403.** In certain embodiments, the near-eye display **403** is see-through to permit the user to view the surrounding environment therethrough. In certain embodiments, the display **403** is configured to selectably display one or more of: a system menu, sighting system **140** menu options, sighting system **140** statistical information such as range, battery power level, user confirmation status, and user biometric data as selected by the user via the optical interface using the camera **400** or the smart glove **256** interface.

The present development allows the system to conform to a single user via dual factor user verification/authentication and allows the operator to control the system without the need for additional devices which slow response time.

The eyewear **400** may be spectacles, goggles, glasses, safety glasses, and so forth. In certain embodiments, the eyewear **412** includes vision corrective lenses. In certain embodiments, the eyewear **412** is configured to protect the user's eyes and the surrounding region from particulates, water, chemicals, and so forth from striking or entering the user's eyes. In certain embodiments, the eyewear **412** includes a lens for filtering one or more wavelengths of electromagnetic radiation. In certain embodiments, the eyewear **412** is configured as sunglasses. In certain embodiments, the eyewear **412** includes polarized lenses.

The eyewear includes a machine-readable indicia **402,** such as a one-dimensional bar code, two-dimensional bar code, QR code, or other optically readable indicia. The bar code or other indicia may appear on a label adhered to the eyewear **412,** or may be embedded, engraved, etched, sandwiched between lens layers, etc., in the eyewear **412.** In certain embodiments, the barcode or other indicia **402** is advantageously positioned at or near the bridge portion of the eyewear, but it will be recognized that the barcode or other indicia may be placed at any other location suitable on the eyewear **412** for imaging by the camera **400,** including without limitation the lens portion of the frame portion of the eyewear.

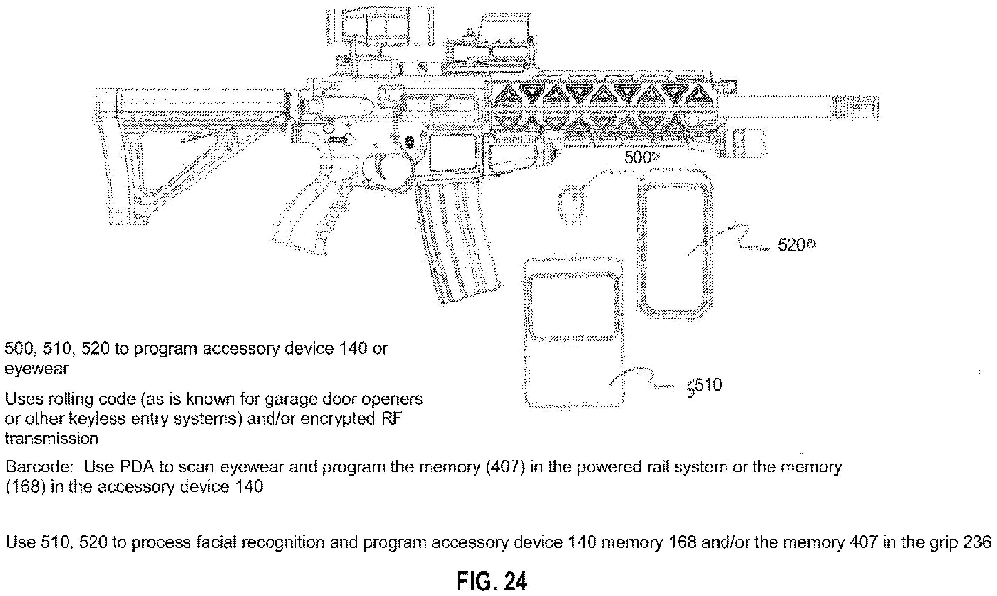

In operation, the camera **400** reads the indicia **402** of the eyewear **412,** which encodes information that serves to identify the user and can be used to retrieve the prior history of use of the system of the user (or of multiple users). In certain embodiments, the user history/data is output to the eyewear display **403.** In certain embodiments, the user history/data is output to the display **176.** In certain embodiments, the user history/data is output to an external device such as portable computing device **510** or smartphone **520** (see FIG. 24), or other storage medium. In certain embodiments, the identified user's data settings (e.g., data settings **325** stored in the memory **168)** are used to configure the device **140** and, optionally, other attached components of the weapon system, with the user's preferences or stored settings. In certain embodiments, the eyewear **412** with barcode **402** serves as a possession-type of discriminator or authentication factor to unlock or otherwise permit the user to operate the weapon system.

In certain embodiments, biometric data may be used, either in addition to or as an alternative to, the barcode/indicia **402** for the purpose of identifying the user, retrieving user data and history, configuring the weapon system, and/or permitting access to or unlocking the weapon system. In embodiments employing biometric data, such data may be facial recognition data or ocular data, such as iris recognition data, eye vein verification data, or retinal scan data. In this manner, the facial recognition data and/or ocular or retinal scan data serve as an inherence type discriminator or authentication factor to unlock or otherwise permit the user to operate the weapon system. In certain embodiments, the user's facial features, retina, and/or ocular features are imaged by the camera **400.**

In alternative embodiments, a camera-equipped smart phone **520** or other mobile or hand-held computing device **510** may be used to image one or more of the bar code/indicia **402,** user's retina, or user's facial features for identification of the user. Imaged data from the device **510** or **520** may be transmitted to the accessory device **140** for processing by the processor **164** or, alternatively, for processing by a processor of the smartphone **520** or portable computing device **510.** In certain embodiments, data is transmitted between the powered rail system and the devices **500, 510, 520** via the remote power supply/battery box **236** as described above.

In certain embodiments, the eyewear **412** is employed in lieu of the glove **256** equipped with RFID **260** described above. In other embodiments, the eyewear **412** is employed in combination with the glove **256** equipped with RFID **260** described above.

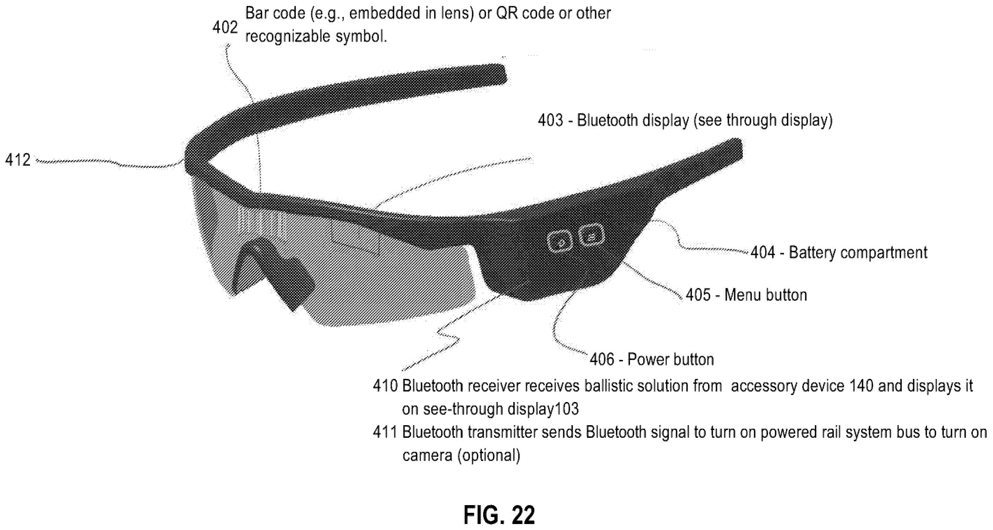

FIG. 22 is an enlarged view of the eyewear **412** illustrating the barcode/indicia **402** on/in the lens portion thereof. In certain embodiments, the eyewear **412** includes a transparent or translucent display **403** which is viewable within the user's field of view when wearing the eyewear **412.** A housing on one of the eyewear temple pieces encloses the associated display driver electronics for driving the display **403** as well as an RF (e.g., Bluetooth) receiver and transceiver pair **410, 411.** A battery compartment **404** is also contained within the temple piece housing for receiving a battery power supply for powering the display and the Bluetooth receiver and transceiver. One or more keys or buttons are also provided on the housing for controlling operation of the display system. In certain embodiments, the buttons/keys include a power button **406** for powering the eyewear **412** on and a menu button **405** for navigating an onscreen menu interface or other on-screen graphical user interface. In certain embodiments, the camera **400** tracks the user's eye movements, to determine what the operator is viewing during operation. In certain embodiments, the camera **400** provides an alternative input means using eye tracking information to control operation of the powered rail system and/or the sighting system **140,** e.g., by navigating a menu-based on-screen user interface, without the need to use input keys or buttons, scroll wheels, etc.

In operation, the Bluetooth receiver **410** receives ballistic solution from the accessory device **140** and displays it on the display **403.** In certain embodiments, the Bluetooth transmitter **411** and receiver **410** are paired with the Bluetooth transceiver **253** in the handgrip **236** and the ballistic solution information is received by the Bluetooth transceiver module **253** via the powered rail system bus **126,** which, in turn, transmits the ballistic solution to the Bluetooth receiver **410** for output to the see-through display **403.**

In certain, optional, embodiments, the Bluetooth transmitter **411** sends a signal to the device **140** via the Bluetooth module **253** to turn on the powered rail system bus **126** and, in turn, turn on the camera **400** to identify the user, e.g., by scanning the barcode/indicia **402,** scanning the user's facial features, and/or scanning the user's retina. In alternative embodiments, the user is verified using the fob **500,** smartphone **520,** or other computing device **510.** In certain embodiments, one or more of the units **500, 510, 520** can be used to identify the user and thereby serve as a possession-type of authentication factor. After the user's identity is determined, the accessory device **140** and/or eyewear **412** can be programed using stored user data and user history in the memory **107,** memory **168,** and/or a memory of the unit **510** or **520**. In certain embodiments, one of the units **510, 520** can be used to program the accessory device **140** and/or eyewear **412** via an application program and user interface on the device **510, 520**.

In certain embodiments, wireless communications between devices such as between the fob **500** and the remote power supply **136** as well as Bluetooth communications between the power supply **136,** grip **236,** and eyewear **412** is encrypted. In certain embodiments, wireless communications may use rolling code security as is known in connection with garage door openers or other keyless entry systems, although other methods of encrypting RF transmissions are also contemplated. In certain embodiments, the PDA **510** or smartphone **520** is used to scan the eyewear **412** and program the memory **407** in the powered rail system and/or the memory **168** in the accessory device **140.** In certain embodiments, the units **510** or **520** are used to process facial recognition and program the memory **168** in the accessory device **140** and/or the memory **407** in the grip **236.**

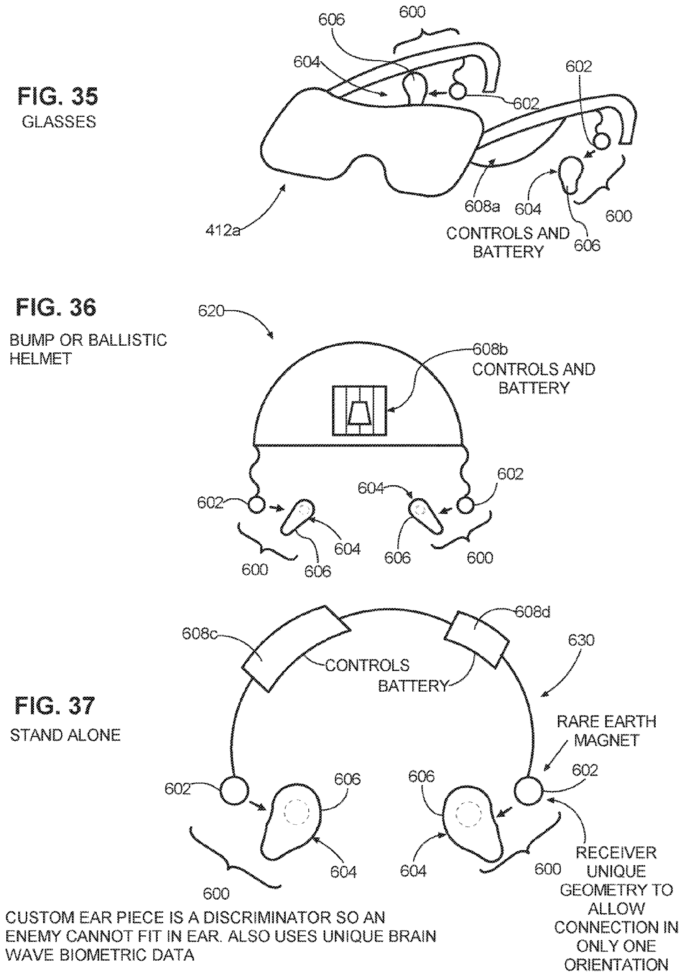

In a further aspect, with reference now to FIGS. 35-37, the present development relates to an in-ear communications apparatus which provides a communications function and is customized to also provide a discrimination function to prevent an enemy or unauthorized person from using the apparatus. Although the illustrated embodiments depict two-ear systems, it will be recognized that systems for use in a single ear are also contemplated, e.g., wherein one of the earpieces is omitted or is replaced with a passive hearing protection device.

The in-ear communications apparatus herein include left and right earpieces **600,** each earpiece **600** including an electrical connector **602** removably attached to an ear-fitting unit **604.** The connector **602** and ear-fitting unit **604** preferably have a have a keyed geometry to allow the devices to connect in one way. In certain embodiments, small but powerful magnets (e.g., rare earth magnets), are employed to secure the electrical connector **602** to the ear-fitting unit **604.** In certain embodiments, each earpiece **600** has a pair of aligned opposing magnets. In other embodiments, either one of the connector **602** or the ear-fitting unit **604** has a magnet and the other has a piece of magnetically attractive material.

The connectors **602** are coupled to a control unit **608** via cables **610** which provide power, data, and signal transfer between the earpieces **600** and the battery and processing electronics within the control unit **608.** The connector **602** will automatically connect to the ear-fitting unit **604** due to the geometrically keyed configuration and the magnetic attraction between the two components. In the event the connector **602** and ear-fitting unit **604** become temporarily separated, e.g., when pulled apart due to being snagged, they will tend to immediately reconnect due to the magnetic attraction between the magnetic fastener elements on the connector **602** and ear-fitting unit **604.** In certain embodiments, the cable components **610** have a length and/or shape that cause them to remain within the range of magnetic attraction between the magnetic fastener elements when the ear fitting units **604** are in the ears of the user and the spectacles **412a,** helmet **620** or headset **630** are donned by the user.

The ear fitting units **604** may have an internal power supply, an external power supply, or preferably both. Because of the limited size of the earplug **604,** it is limited as to the size of the power supply it can accommodate internally. The external control module **608** can accommodate a much larger battery to allow the earplug units to operate for long period of time.

Each ear-fitting unit **604** includes an exterior enclosure **606** which is custom fitted to the precise shape of a particular user's ear to be worn in the pinna/ear canal region of the user's ear. The enclosure **606** houses an audio speaker, microphone, and one or more biological sensors, which may be biomedical or biometric sensors.

In certain embodiments, the biological sensors may include electrodes and associated amplifiers for measuring electrical signals of the body, including without limitation EEG signals from the brain and EKG/ECG signals from the heart. For example, EEG signals can be used to determine sleep or waking state. EKG/ECG signals can be used to determine heart rate, heart rate variability, identify heart rhythm characteristics, abnormalities, or other information. Temperature transducers may be provided for measuring temperature of the body. Other sensors include moisture sensors, which may be used to monitor sweating by the user. Optical sensors are also contemplated. Exemplary optical sensors may be used for sensing heart rate and blood oxygenation and other biomedical information. It will be recognized that the biological signals may be used for medical monitoring of the user as well as for biometric identification and authentication of the user.

The custom fit nature of the ear-fitting units **604, 606** serves as a first level discriminator for preventing an enemy or other unauthorized user from using the ear pieces in accordance with this disclosure because it is custom fit and, therefore, unlikely to fit into another user's ear. However, in preferred embodiments, biometric data sensed by the earpieces is used to identify the user and provide an inherency based authentication factor. In a preferred embodiment, biometric data related to the user's unique brain wave signals can be used to identify and authenticate the user.

The speaker, microphone, and one or more sensors are electrically coupled to a control module 608, which houses signal processing circuitry for receiving signals from the microphone and biological sensors and outputting an audio signal to the speaker. The speaker and microphone can be used for audio communication purpose. In certain embodiments, the speaker and microphone can also be used for biometric identification and authentication. For example, the speaker and microphone can be used to detect the acoustic properties or acoustic signature of the user's ear canal, which are unique to each individual.

In certain embodiments, the signal processing circuitry includes analog-to-digital and digital-to-analog conversion circuitry, which may be dedicated hardware ADCs and DACs, or, may be implemented in software or firmware in a microprocessor or microcontroller. The control module **608** also includes an RF (e.g., Bluetooth) transceiver for two-way wireless communication with an associated device or network. In certain embodiments, the RF transceiver is a Bluetooth receiver configured for communications with the Bluetooth transceiver **222** of the remote power supply **136** and/or the Bluetooth transceiver **253** of the firearm grip **236.** The control module **608** also includes a battery compartment for receiving a battery power supply for powering operation of the wireless transceiver units and the signal processing circuitry.

The audio speaker component of the earpieces **600** can be used for audio communication purposes, hands free telephone usage, listening to audio, and so forth. The earpieces **600** may also employ a noise cancellation function to reduce background noise. In certain embodiments, the audio speaker component is used in conjunction with an external microphone to convert external sounds to an audio signal to the audio speakers of the earpieces **600.** In certain embodiments, the external microphone is wireless microphone for transmitting audio information to the earpieces via the Bluetooth receiver in the control unit **608.** In certain embodiments, the external microphone is a directional microphone or microphone array for recording both sound and directional information of the sound. In certain embodiments, the processing electronics are configured to process the audio signal along with the directional information using a three-dimensional, binaural, or head-related transfer function (HRTF) algorithm sound processing algorithm which allows the user to interpret the sounds output by the audio speaker as coming from specific directions or points in space.

In certain embodiments, the sound processing electronics is configured to identify potentially threatening sounds and warn the user audibly and/or visually. An example of an audible warning might be a synthesized or prerecorded voice saying "enemy intruder approaching at 4 o'clock." Alternatively or additionally, if the user is employing the eyewear **412a** (see FIG. 35) or **412** (see, e.g., FIG. 22), a visual warning may appear in the display screen **403.** If the user is not using the eyewear **412** or **412a,** a visual warning may be output to another display associated with the weapon system, such as the display **176.** In certain embodiments, the earpieces **600** can be used for transmitting weapon settings and emergency settings via the Bluetooth interface in the control module **608.**

Referring now to FIG. 35, there appears eyewear **412a,** which include the ear pieces **600** as detailed above, configured to be worn when the user is wearing the glasses **412a,** and which may be otherwise similar to the eyewear **412** discussed above by way of reference to FIGS. 21-25. The aforementioned control circuitry, transceiver, and power supply are housed in the housing **608a** located on the temple piece. Referring now to FIG. 36, there appears a piece of headwear **620** such as a military bump helmet, ballistic helmet, or the like. The headwear **620** includes earpieces **600** configured to be worn by the user when the helmet is worn. The aforementioned control circuitry, transceiver, and power supply are housed in the housing **608b** located on the helmet. For example, the housing **608b** may be attached to a rear connector on the helmet, or, incorporated into or attached to a shroud located on the front of the helmet **620.** It will be recognized that other methods for attaching a power supply and electronics to a helmet are also contemplated.

Referring now to FIG. 37, there appears a standalone communications headset **630** comprising the earpieces **600** as detailed above and having a band, strap, or cable which extends over or behind the user's head. In the illustrated embodiment, the control circuitry and transceiver are housed within a control module housing **608c** and the battery is housed within a battery housing **608d.**

In certain embodiments, the earpieces **600** provide a brain computer interface for controlling operation of the associated weapon system. The brain wave signals from the electrodes in the earpiece **604** are sent to the signal processing electronics in the control module **608** and analyzed for a desired physiological movement or function. In this manner, the user can consciously manipulate his or her own brain activity, which is reflected in the brain wave signal transmitted to the processing electronics **608.** The brain wave signal is monitored by the processing electronics until a signal or pattern representative of an operation or function to be performed on or by the weapon system disclosed is identified. If a brain wave signal or pattern that is representative of an operation or function to be performed on or by the weapon system, the processor in the processing electronics **608** generates an appropriate control signal which is transferred via the RF transmitter within the control unit **608** to a receiver such as transceiver **222** or **253.** The control signal is then transferred via the bus **126** to a device coupled to the data bus **126.**

For example, the operator can control the weapon system by consciously thinking of taking a certain action, e.g., turning on the flashlight **188** at 25% intensity. In certain embodiments, the use of brain wave signals to control the present weapon system may be provided in addition to dedicated buttons, switches, keypads, and for forth provided on the weapon system that can be manipulated manually to control operation of the weapon system herein. In other embodiments, however, it is contemplated that the number of dedicated keypads, buttons, switches, etc. can be reduced or eliminated in favor of the brain-computer interface provided by the earpieces **604.** In certain embodiments, the weapon system herein is configured to restrict use to a particular individual or individuals.

As another example, it has been found that humans, such as a well-trained soldier, are capable of visually estimating a distance to a target with a reasonable degree of accuracy (e.g., +/- 25 meters). Such estimates are suitable for a number of targeting applications. In this manner, the user can consciously manipulate his or her brain activity based on an estimated distance, which is reflected in the brain wave signal monitored by the processing electronics **608.** A brain wave signal representative of estimated distance to a target is converted by the processing electronics **608** into an appropriate control signal which is transferred via the RF transmitter within the control unit **608** to a receiver such as transceiver **222** or **253.** The control signal is then transferred via the bus **126** to a device coupled to the data bus **126.** For example, the operator may automatically adjust the trajectory system of the reflex sight **160** by consciously estimating the target distance. As another example, a brain wave signal of approximate distance may be used to adjust a variable power scope to the proper power to engage an enemy. In certain embodiments, the use of brain wave signals for range estimation may be a functionality provided in addition to a dedicated range finder such as an optical range finder. In other embodiments, however, it is contemplated a range finding function implemented via the brain-computer interface function of the earpieces **600** would eliminate the need to have a rangefinder on the weapon.

The invention has been described with reference to the preferred embodiments. Obviously, modifications and alterations will occur to others upon reading and understanding the preceding detailed description. It is intended that the invention be construed as including all such modifications and alterations, insofar as they come within the scope of the appended claims.

## Claims

1. A weapon system for use with a firearm, comprising:
a processor (164), the processor (164) having an associated memory (168), the processor (164) configured to receive sensor data from one or more sensors, the sensor data selected from the group consisting of:
temperature data representative of a temperature of a barrel of the firearm;
round count data representative of a number of rounds of ammunition fired by the firearm;
barrel life expectancy data representative of wear accumulated by the barrel of the firearm;
rate of fire data representative of a number of rounds of ammunition fired by the firearm per unit time;
projectile velocity data representative of a velocity of the rounds of ammunition fired by the firearm;
ammunition type data representative of a type of ammunition fired by the firearm;
barrel whip data representative of one or more harmonic characteristics of the barrel of the firearm; and
any combination of the foregoing;
an optical reader (400), the optical reader (400) configured to receive operator-identification information and transmit the operator-identification information to the processor (164); and
a sighting system (140) operably coupled to the processor (164), the sighting system (140) including one or more aiming lasers (148, 152), wherein the processor (164) is configured to perform ballistics calculations and automatically adjust an angle of the one or more aiming lasers (148, 152) to improve accuracy of the sighting system (140) based on said sensor data.

2. The weapon system of claim 1, wherein the optical reader (400) is a camera configured to generate image signals.

3. The weapon system of claim 2, wherein the optical reader (400) is configured to detect optically readable indicia.

4. The weapon system of claim 3, wherein the optically readable indicia is a bar code.

5. The weapon system of claim 2, wherein the camera comprises a facial-recognition system responsive to the image signals generated by the camera.

6. The weapon system of claim 5, wherein the facial recognition system is controllably coupled to the weapon system.

7. The weapon system of claim 2, wherein the operator-identification information is selected from the group consisting of facial feature data, iris recognition data, eye vein verification data, ocular feature data, machine-readable indicia.

8. The weapon system of claim 2, wherein the camera is capable of tracking one or both of eye movement of a user and positioning of at least one eye of the user in relation to the firearm.

9. The weapon system of claim 1, further comprising an accessory eyewear device (412) configured for optical communication with the optical reader (400).

10. The weapon system of claim 9, wherein the accessory eyewear device (412) comprises a machine-readable indicia (402).

11. The weapon system of claim 10, wherein the machine-readable indicia (402) is selected from the group consisting of: one dimensional bar code, two-dimensional bar code, and Quick Response (QR) code.

12. The weapon system of claim 10, wherein the optical reader (400) is a camera capable of reading the machine-readable indicia (402).

13. The weapon system of claim 9, wherein the accessory eyewear device (412) comprises a near-eye display screen (403).

14. The weapon system of claim 13, wherein the near-eye display screen (403) is configured to display one or more of the following: a system menu, sighting system menu options, sighting system statistical information, battery power level, user confirmation status, and user biometric data.

15. The weapon system of claim 9, the accessory eyewear device (412) further comprising an in-ear communication system, the in-ear communication system (600) comprising an electrical connector (602) and an ear-fitting unit (604) removably attachable to the electrical connector (602).

## Patentansprüche

1. Waffensystem zur Verwendung mit einer Feuerwaffe, umfassend:
einen Prozessor (164), wobei der Prozessor (164) einen zugeordneten Speicher (168) aufweist, wobei der Prozessor (164) konfiguriert ist, um Sensordaten von einem oder mehreren Sensoren zu empfangen, wobei die Sensordaten aus der Gruppe ausgewählt sind, bestehend aus:
Temperaturdaten, die repräsentativ für eine Temperatur eines Laufs der Feuerwaffe sind;
Schusszahldaten, die repräsentativ für eine Anzahl von durch die Feuerwaffe abgefeuerten Munitionsschüssen sind;
Lauflebenserwartungsdaten, die repräsentativ für einen durch den Lauf der Feuerwaffe angesammelten Verschleiß sind;
Feuerratendaten, die repräsentativ für eine Anzahl von durch die Feuerwaffe pro Zeiteinheit abgefeuerten Munitionsschüssen sind;
Projektilgeschwindigkeitsdaten, die repräsentativ für eine Geschwindigkeit der durch die Feuerwaffe abgefeuerten Munitionsschüsse sind;
Munitionsartdaten, die repräsentativ für eine Art von durch die Feuerwaffe abgefeuerter Munition sind;
Laufschwingungsdaten, die repräsentativ für eine oder mehrere harmonische Eigenschaften des Laufs der Feuerwaffe sind; und
jeder beliebigen Kombination der Vorstehenden;
ein optisches Lesegerät (400), wobei das optische Lesegerät (400) konfiguriert ist, um Bedienererkennungsinformationen zu empfangen und die Bedienererkennungsinformationen an den Prozessor (164) zu übertragen; und
ein Visiersystem (140), das wirkfähig mit dem Prozessor (164) gekoppelt ist, wobei das Visiersystem (140) einen oder mehrere Ziellaser (148, 152) beinhaltet, wobei der Prozessor (164) konfiguriert ist, um Ballistikberechnungen durchzuführen und automatisch einen Winkel des einen oder der mehreren Ziellaser (148, 152) anzupassen, um eine Genauigkeit des Visiersystems (140) basierend auf den Sensordaten zu verbessern.

2. Waffensystem nach Anspruch 1, wobei das optische Lesegerät (400) eine Kamera ist, die konfiguriert ist, um Bildsignale zu erzeugen.

3. Waffensystem nach Anspruch 2, wobei das optische Lesegerät (400) konfiguriert ist, um optisch lesbare Markierungen zu erfassen.

4. Waffensystem nach Anspruch 3, wobei die optisch lesbaren Markierungen ein Strichcode sind.

5. Waffensystem nach Anspruch 2, wobei das Gesichtserkennungssystem auf die von der Kamera erzeugten Bildsignale reagiert.

6. Waffensystem nach Anspruch 5, wobei das Gesichtserkennungssystem steuerbar mit dem Waffensystem gekoppelt ist.

7. Waffensystem nach Anspruch 2, wobei die Bedienererkennungsinformationen aus der Gruppe ausgewählt sind, die besteht aus Gesichtsmerkmalsdaten, Iriserkennungsdaten, Augenvenenverifizierungsdaten, Augenmerkmalsdaten, maschinenlesbaren Markierungen.

8. Waffensystem nach Anspruch 2, wobei die Kamera in der Lage ist, eine oder beide von einer Augenbewegung eines Benutzers und einer Positionierung von zumindest einem Auge des Benutzers in Bezug auf die Feuerwaffe zu verfolgen.

9. Waffensystem nach Anspruch 1, ferner umfassend eine Zubehör-Brillenvorrichtung (412), die für eine optische Kommunikation mit dem optischen Lesegerät (400) konfiguriert ist.

10. Waffensystem nach Anspruch 9, wobei die Zubehör-Brillenvorrichtung (412) maschinenlesbare Markierungen (402) umfasst.

11. Waffensystem nach Anspruch 10, wobei die maschinenlesbaren Markierungen (402) aus der Gruppe ausgewählt sind, bestehend aus: eindimensionalem Strichcode, zweidimensionalem Strichcode und Quick-Response-(QR-)Code.

12. Waffensystem nach Anspruch 10, wobei das optische Lesegerät (400) eine Kamera ist, die in der Lage ist, die maschinenlesbare Markierungen (402) zu lesen.

13. Waffensystem nach Anspruch 9, wobei die Zubehör-Brillenvorrichtung (412) einen augennahen Anzeigebildschirm (403) umfasst.

14. Waffensystem nach Anspruch 13, wobei der augennahe Anzeigebildschirm (403) konfiguriert ist, um eines oder mehrere der Folgenden anzuzeigen: ein Systemmenü, Visiersystem-Menüoptionen, statistische Visiersysteminformationen, Batterieleistungspegel, Benutzerbestätigungsstatus und biometrische Benutzerdaten.

15. Waffensystem nach Anspruch 9, wobei die Zubehör-Brillenvorrichtung (412) ferner ein In-Ear-Kommunikationssystem umfasst, wobei das In-Ear-Kommunikationssystem (600) einen elektrischen Verbinder (602) und eine ans Ohr passbare Einheit (604) umfasst, die abnehmbar an dem elektrischen Verbinder (602) anbringbar ist.

## Revendications

1. Système d'arme destiné à être utilisé avec une arme à feu, comprenant :
un processeur (164), le processeur (164) ayant une mémoire associée (168), le processeur (164) étant configuré pour recevoir des données de capteurs provenant d'un ou plusieurs capteurs, les données de capteurs étant sélectionnées dans le groupe constitué :
de données de température représentatives de la température du canon de l'arme à feu ;
de données de comptage des coups représentatives du nombre de cartouches tirées par l'arme à feu ;
de données sur la durée de vie du canon représentatives de l'usure accumulée par le canon de l'arme à feu ;
de données sur la cadence de tir représentatives du nombre de cartouches tirées par l'arme à feu par unité de temps ;
de données de vitesse du projectile représentatives de la vitesse des cartouches tirées par l'arme à feu ;
de données sur le type de cartouches représentatives d'un type de cartouches tirées par l'arme à feu ;
de données relatives au fouettement du canon représentatives d'une ou plusieurs caractéristiques harmoniques du canon de l'arme à feu ; et
de toute combinaison des éléments précédents ;
d'un lecteur optique (400), le lecteur optique (400) étant configuré pour recevoir des informations d'identification de l'opérateur et transmettre ces informations au processeur (164) ; et
d'un système de visée (140) couplé de manière fonctionnelle au processeur (164), le système de visée (140) comportant un ou plusieurs lasers de visée (148, 152), dans lequel le processeur (164) est configuré pour effectuer des calculs balistiques et ajuster automatiquement un angle du un ou plusieurs lasers de visée (148, 152) pour améliorer la précision du système de visée (140) sur la base desdites données de capteurs.

2. Système d'arme selon la revendication 1, dans lequel le lecteur optique (400) est une caméra configurée pour générer des signaux d'image.

3. Système d'arme selon la revendication 2, dans lequel le lecteur optique (400) est configuré pour détecter des indices optiquement lisibles.

4. Système d'arme selon la revendication 3, dans lequel les indices optiquement lisibles sont un code-barres.

5. Système d'arme selon la revendication 2, dans lequel la caméra comprend un système de reconnaissance faciale répondant aux signaux d'image générés par la caméra.

6. Système d'arme selon la revendication 5, dans lequel le système de reconnaissance faciale est couplé de manière contrôlée au système d'arme.

7. Système d'arme selon la revendication 2, dans lequel les informations d'identification de l'opérateur sont sélectionnées dans le groupe constitué de données sur les caractéristiques faciales, de données de reconnaissance de l'iris, de données de vérification des veines oculaires, de données sur les caractéristiques oculaires et d'indices lisibles par machine.

8. Système d'arme selon la revendication 2, dans lequel la caméra est capable de suivre un ou les deux mouvements oculaires d'un utilisateur et le positionnement d'au moins un œil de l'utilisateur par rapport à l'arme à feu.

9. Système d'arme selon la revendication 1, comprenant en outre un dispositif de lunettes accessoire (412) configuré pour la communication optique avec le lecteur optique (400).

10. Système d'arme selon la revendication 9, dans lequel le dispositif de lunettes accessoire (412) comprend un indice lisible par machine (402).

11. Système d'arme selon la revendication 10, dans lequel les indices lisibles par machine (402) sont sélectionnés dans le groupe constitué de : un code-barres unidimensionnel, un code-barres bidimensionnel, et un code à réponse rapide (QR).

12. Système d'arme selon la revendication 10, dans lequel le lecteur optique (400) est une caméra capable de lire les indices lisibles par machine (402).

13. Système d'arme selon la revendication 9, dans lequel le dispositif de lunettes accessoire (412) comprend un écran d'affichage proche de l'oeil (403).

14. Système d'arme selon la revendication 13, dans lequel l'écran d'affichage proche de l'œil (403) est configuré pour afficher l'un ou plusieurs des éléments suivants : un menu système, des options de menu du système de visée, des informations statistiques du système de visée, le niveau de charge de la batterie, l'état de confirmation de l'utilisateur et les données biométriques de l'utilisateur.

15. Système d'arme selon la revendication 9, le dispositif de lunettes accessoire (412) comprenant en outre un système de communication intra-auriculaire, le système de communication intra-auriculaire (600) comprenant un connecteur électrique (602) et une unité de fixation auriculaire (604) pouvant être fixée de manière amovible au connecteur électrique (602).
